# EUROPEAN PATENT APPLICATION

(11) **EP 1 632 227 A1**
(43) Date of publication of application: **08.03.2006**
(21) Application number: 04021204.5
(22) Date of filing: 07.09.2004
(51) Int. Cl.: A61K 31/415, A61K 31/4164, A61K 31/4188, A61P 25/30, A61P 25/36

(54) **Derivatives of aryl (or heteroaryl) azolylcarbinols (in particular cizolirtin citrate) for the treatment of opioid addiction**

(71) Applicant: Laboratorios Dr. Esteve S.A., 08041 Barcelona (ES)
(72) Inventor: Farre Gomis, Antonio J. Lab. del Dr. Esteve, S.A., 08041 Barcelona (ES)

(57) **Abstract**

The present invention refers to the use of derivatives of aryl (or heteroaryl) azolylcarbinols of general formula (I), and their physiologically acceptable salts, as medicinal products for human and/or animal therapeutics for the treatment of the symptoms of central neuropathic pain, especially certain subtypes of central neuropathic pain, as well as treatment of the disease causing the symptoms, the prevention or the prophylaxis of the symptoms of central neuropathic pain, especially certain subtypes of central neuropathic pain, as well as the prevention or the prophylaxis of the disease causing the symptoms. The preferred compound is Cizolirtin Citrate.

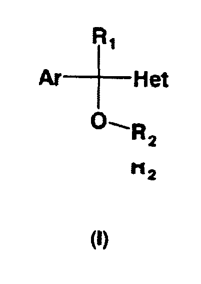

## Description

### Field of the invention

The present invention refers to the use of derivatives of aryl (or heteroaryl) azolylcarbinols of general formula (I), and their physiologically acceptable salts, as medicinal products for human and/or animal therapeutics for the treatment of opioid addiction.

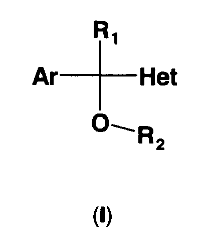

### Background of the invention

Addiction especially from opioids like morphine can by all rights nowadays be called a mass disease.

Therefore, it was the underlying problem solved by this invention to find new ways of treating opioid addiction.

The patents EP 289380 B1 (US 5,017,596) and WO 99/52525 (US 6,410,582) describe derivatives of carbinols of general formula (I) with analgesic activity

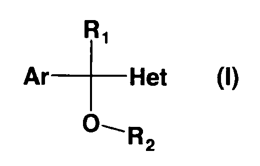

In EP 289380 B1 (US 5,017,596) and WO 99/52525 (US 6,410,582) these compounds of general formula (I). Ar represents a benzene ring or a thiophene ring with or without substitutions, R₁ represents a hydrogen atom or a lower alkyl group from C₁ to C₄; R₂ represents a dialkylaminoalkyl or azaheterocyclylalkyl and Het represents an azole with or without substitutions, and their physiologically acceptable salts.

In EP 1103243/US 6,518,295 the use of carbinols of general formula (I) for the treatment of excess of substance P and diseases mediated by that excess (including drug addiction) has been described. There are also other different applications covering the use of these carbinols for different indications (EP 1 086 682 (WO99/59524); EP 1 384 476 (WO02/080 908); EP 1 413 305/US2003/0022925).

In the patent applications WO 97/20817 (US 5,849,931), WO 99/02500 (US 6,187,930), WO 99/07684 (US 6,118,009) and WO 99/52525 (US 6,410,582) there are also described several procedures to prepare enantiomerically pure compounds with general formula (I).

Testing the compounds it was quite impressing that compounds of general formula (I), and their physiologically acceptable salts, were so effective in tests of their ability to provide a relieve from opioid addiction.

### Detailed description of the invention

The present invention refers to the use of a carbinol compounds of general formula (II) 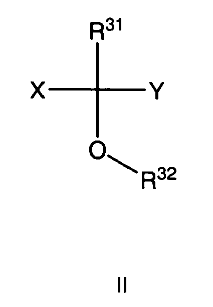 wherein
R³¹ represents a hydrogen atom, a linear or branched alkyl radical, a linear or branched alkenyl radical, an optionally at least mono-substituted cycloaliphatic radical, which may contain at least one nitrogen atom as ring member, or a phenyl radical,
R³² represents a hydrogen atom, an optionally at least one nitrogen atom as ring member containing cycloaliphatic radical, which may be at least mono-substituted by a linear or branched alkyl radical and/or which may be bound via a linear or branched alkylene group, an NR³³R³⁴⁻moiety, which is bound via a linear or branched alkylene group, or an NR³⁵R³⁶-moiety, which is bound via a linear or branched alkylene group,
R³³ and R³⁴, identical or different, represent hydrogen, a linear or branched alkyl radical or an unsubstituted benzyl radical,
R³⁵ and R³⁶ together with the bridging nitrogen atom represent a saturated, unsubstituted, optionally at least one further heteroatom as ring member containing heterocyclic radical,
X represents an optionally at least mono-substituted phenyl radical or an optionally at least mono-substituted thienyl radical, wherein in each case the substituents may be independently selected from the group consisting of a linear or branched alkyl radical, a linear or branched alkoxy group, a linear or branched alkyl radical, which is at least partially halogenated and a halogen atom,
Y represents a heteroaryl radical, which contains one or more nitrogen atoms as ring members and which is not substituted or at least mono-substituted by one or more substitutents independently from one another selected from the group consisting of a halogen atom, a linear or branched alkyl radical, a benzyl radical, a ciano group bound via a linear or branched C₁₋₄-alkylene group, a carboxy group bound via a linear or branched C₁₋₄-alkylene group, a methoxy carbonyl group bound via a linear or branched C₁₋₄-alkylene group, a hydroxy group bound via a linear or branched C₁₋₄-alkylene group, an amino group bound via a linear or branched C₁₋₄-alkylene group, a (C₁₋₄) dialkylamino group bound via a linear or branched C₁₋₄-alkylene group, and a cycloaliphatic radical, which contains at least one nitrogen atom as ring member and which is bound via a linear or branched C₁₋₄-alkylene group, or Y represents an unsubstituted heteroaryl radical, which contains two nitrogen atoms as ring members and which is condensed with a saturated, one methyl-substituted nitrogen atom as ring member containing cycloaliphatic group,
optionally in form of one of its stereoisomers, preferably enantiomers or diastereomers, its racemate or in form of a mixture of at least two of its stereoisomers, preferably enantiomers and/or diastereomers, in any mixing ratio, or a corresponding salt thereof, or a corresponding solvate
for the manufacture of a medicament for drug addiction treatment from addiction to opioids, for the treatment of drug abuse of opioids, for the treatment of dependency from opioids or to ameliorate the effects of a de-addiction treatment from addiction to opioids.

"Drug Addiction treatment from addiction to opioids" is defined generally as the processes used to reduce (possibly up to zero) the amount of opioid the patient needs or is consuming. "Treatment of drug abuse of opioids" is defined as the medical therapeutic approaches to stop the abuse of this opioid or any opioid in general. This also includes detoxification and any replacement therapy of the opioid or similar approaches. "Treatment of dependency from opioids" is defined as the medical therapeutic approaches to lessen the dependency of a patient on this opioid or any opioid in general having the aim of reducing all dependency at all, whereas "dependency" is defined as being either psychologically or more prominently physiologically forced to consume the opioid. The term "effects of a de-addiction treatment" in the context of this invention is understood as including any side effect coming with any de-addiction treatment, especially any kind of withdrawal syndrome, whereas "ameliorating" or "amelioration" means bettering the conditions for the patient either objectively or subjectively, like less or less felt withdrawal syndromes.

"Addiction" is defined as the compulsive uncontrolled use of habitforming drugs (opioids) beyond the period of medical need (Webster's Third New International Dictionary, 1993).

It is especially preferred if the addiction is not a disorder mediated by excess of substance P or it is preferred if the addiction is not a disorder connected to an excess of substance P.

Opioids such as morphine, which belong to the class of centrally acting analgesics have a high risk of addiction. For the purposes of the present invention the term opioid includes substances having affinity for one or more of the opioid receptors such as the µ-opioid receptors, the δ-opioid receptors and/or the κ-opioid receptors.

Especially this invention refers to those opioids, which act as agonists or partial agonists on these receptors and who naturally also develop addiction. Examples include heroine and morphine and their analogues but also other opioids.

In the context of this invention, alkyl and cycloalkyl radicals are understood as meaning saturated and unsaturated (but not aromatic), branched, unbranched and cyclic hydrocarbons, which can be unsubstituted or mono- or polysubstituted. In these radicals, C₁₋₂-alkyl represents C1- or C2-alkyl, C₁₋₃-alkyl represents C1-, C2-or C3-alkyl, C₁₋₄-alkyl represents C1-, C2-, C3- or C4-alkyl, C₁₋₅-alkyl represents C1-, C2-, C3-, C4-, or C5-alkyl, C₁₋₆-alkyl represents C1-, C2-, C3-, C4-, C5- or C6-alkyl, C₁₋₇-alkyl represents C1-, C2-, C3-, C4-, C5-, C6- or C7-alkyl, C₁₋₈-alkyl represents C1-, C2-, C3-, C4-, C5-, C6-, C7- or C8-alkyl, C₁₋₁₀-alkyl represents C1-, C2-, C3-, C4-, C5-, C6-, C7-, C8-, C9- or C10-alkyl and C₁₋₁₈-alkyl represents C1-, C2-, C3-, C4-, C5-, C6-, C7-, C8-, C9-, C10-, C11-, C12-, C13-, C14-, C15-, C16-, C17- or C18-alkyl. Furthermore, C₃₋₄-cycloalkyl represents C3- or C4-cycloalkyl,

C_{3- 5}-cycloalkyl represents C3-, C4- or C5-cycloalkyl, C₃₋₆-cycloalkyl represents C3-, C4-, C5- or C6-cycloalkyl, C₃₋₇-cycloalkyl represents C3-, C4-, C5-, C6- or C7-cycloalkyl, C₃₋₈-cycloalkyl represents C3-, C4-, C5-, C6-, C7- or C8-cycloalkyl, C₄₋₅-cycloalkyl represents C4- or C5-cycloalkyl, C₄₋₆-cycloalkyl represents C4-, C5- or C6-cycloalkyl, C₄₋₇-cycloalkyl represents C4-, C5-, C6- or C7-cycloalkyl, C₅₋₆-cycloalkyl represents C5- or C6-cycloalkyl and C₅₋₇-cycloalkyl represents C5-, C6- or C7-cycloalkyl. In respect of cycloalkyl, the term also includes saturated cycloalkyls in which one or 2 carbon atoms are replaced by a heteroatom, S, N or O. However, mono- or polyunsaturated, preferably monounsaturated, cycloalkyls without a heteroatom in the ring also in particular fall under the term cycloalkyl as long as the cycloalkyl is not an aromatic system. The alkyl and cycloalkyl radicals are preferably methyl, ethyl, vinyl (ethenyl), propyl, allyl (2-propenyl), 1-propinyl, methylethyl, butyl, 1-methylpropyl, 2-methylpropyl, 1,1-dimethylethyl, pentyl, 1,1-dimethylpropyl, 1,2-dimethylpropyl, 2,2-dimethylpropyl, hexyl, 1-methylpentyl, cyclopropyl, 2-methylcyclopropyl, cyclopropylmethyl, cyclobutyl, cyclopentyl, cyclopentylmethyl, cyclohexyl, cycloheptyl, cyclooctyl, and also adamantyl, (if substituted also CHF₂, CF₃ or CH₂OH) as well as pyrazolinone, oxopyrazolinone, [1,4]-dioxane or dioxolane.

Here, in connection with alkyl and cycloalkyl - unless expressly defined otherwise - the term substituted in the context of this invention is understood as meaning replacement of at least one hydrogen radical by F, Cl, Br, I, NH₂, SH or OH, "polysubstituted" radicals being understood as meaning that the replacement takes effect both on different and on the same atoms several times with the same or different substituents, for example three times on the same C atom, as in the case of CF₃, or at different places, as in the case of -CH(OH)-CH=CH-CHCl₂. Particularly preferred substituents here are F, Cl and OH. In respect of cycloalkyl, the hydrogen radical can also be replaced by OC₁₋₃-alkyl or C₁₋₃-alkyl (in each case mono- or polysubstituted or unsubstituted), in particular methyl, ethyl, n-propyl, i-propyl, CF₃, methoxy or ethoxy.

The term (CH₂)₃₋₆ is to be understood as meaning -CH₂-CH₂-CH₂-, -CH₂-CH₂-CH₂₋CH₂-, -CH₂-CH₂-CH₂-CH₂-CH₂- and -CH₂-CH₂-CH₂-CH₂-CH₂-CH₂-, (CH₂)₁₋₄ is to be understood as meaning -CH₂-, -CH₂-CH₂-, -CH₂-CH₂-CH₂- and -CH₂-CH₂-CH₂-CH₂-, (CH₂)₄₋₅ is to be understood as meaning -CH₂-CH₂-CH₂-CH₂- and -CH₂-CH₂-CH₂₋CH₂-CH₂-, etc.

An aryl radical is understood as meaning ring systems with at least one aromatic ring but without heteroatoms even in only one of the rings. Examples are phenyl, naphthyl, fluoranthenyl, fluorenyl, tetralinyl or indanyl, in particular 9H-fluorenyl or anthracenyl radicals, which can be unsubstituted or monosubstituted or polysubstituted.

A heteroaryl radical is understood as meaning heterocyclic ring systems which have at least one unsaturated ring and can contain one or more heteroatoms from the group consisting of nitrogen, oxygen and/or sulfur and can also be mono- or polysubstituted. Examples which may be mentioned from the group of heteroaryls are furan, benzofuran, thiophene, benzothiophene, pyrrole, pyridine, pyrimidine, pyrazine, quinoline, isoquinoline, phthalazine, benzo-1,2,5-thiadiazole, benzothiazole, indole, benzotriazole, benzodioxolane, benzodioxane, carbazole and quinazoline.

Here, in connection with aryl and heteroaryl, substituted is understood as meaning substitution of the aryl or heteroaryl by R, OR, a halogen, preferably F and/or Cl, a CF₃, a CN, an NO₂, an NRR, a C₁₋₆-alkyl (saturated), a C₁₋₆₋alkoxy, a C₃₋₈₋cycloalkoxy, a C₃₋₈-cycloalkyl or a C₂₋₆-alkylene.

The term "salt" is to be understood as meaning any form of the active compound used according to the invention in which it assumes an ionic form or is charged and is coupled with a counter-ion (a cation or anion) or is in solution. By this are also to be understood complexes of the active compound with other molecules and ions, in particular complexes which are complexed via ionic interactions.

The term "physiologically acceptable salt" means in the context of this invention any salt that is physiologically tolerated (most of the time meaning not being toxic-especially not caused by the counter-ion) if used appropriately for a treatment especially if used on or applied to humans and/or mammals.

These physiologically acceptable salts can be formed with cations or bases and in the context of this invention is understood as meaning salts of at least one of the compounds used according to the invention - usually a (deprotonated) acid - as an anion with at least one, preferably inorganic, cation which is physiologically tolerated - especially if used on humans and/or mammals. The salts of the alkali metals and alkaline earth metals are particularly preferred, and also those with NH4, but in particular (mono)- or (di)sodium, (mono)- or (di)potassium, magnesium or calcium salts.

These physiologically acceptable salts can also be formed with anions or acids in the context of this invention is understood as meaning salts of at least one of the compounds used according to the invention - usually protonated, for example on the nitrogen - as the cation with at least one anion which are physiologically tolerated - especially if used on humans and/or mammals. By this is understood in particular, in the context of this invention, the salt formed with a physiologically tolerated acid, that is to say salts of the particular active compound with inorganic or organic acids which are physiologically tolerated - especially if used on humans and/or mammals.
Examples of physiologically tolerated salts of particular acids are salts of: hydrochloric acid, hydrobromic acid, sulfuric acid, methanesulfonic acid, formic acid, acetic acid, oxalic acid, succinic acid, malic acid, tartaric acid, mandelic acid, fumaric acid, lactic acid or citric acid.

The term "solvate" according to this invention is to be understood as meaning any form of the active compound according to the invention in which this compound has attached to it via non-covalent binding another molecule (most likely a polar solvent) especially including hydrates and alcoholates, e.g. methanolate.

The compounds of general formula (II) (as well as I, Ia, Ib and Ic) can be synthesised according to the procedures described in patents EP 289380, US 5,017,596 or WO 99/52525. The compounds of general formula (II) (as well as I, la, Ib and Ic) have a stereogenic centre and the invention refers both to the use of a pure enantiomer and to the use of a mixture of enantiomers. The enantiomers can be prepared by any of the procedures described in our patents WO 97/20817 (US 5,849,931), WO 99/02500 (US 6,187,930), WO 99/07684 (US 6,118,009) and WO 99/52525 (US 6,410,582).

In the context of this invention the term "(dimethylamino)" shall be treated and considered absolutely identical to the term "(dimethylamine). The selection of the first term to describe compounds was only due a seeming more fitting chemical nomenclature. Also, for the sake of clarity it is stated here that the expression "thienyl" is for this invention one and the same as "thiophenyl" and shall also be treated and considered as absolutely identical to each other.

In a preferred aspect of the invention the compound according to formula II used is characterized in that R³¹ represents a hydrogen atom, a linear or branched C₁₋₄ alkyl radical, a linear or branched C₂₋₄ alkenyl radical, a 5- or 6-membered cycloaliphatic radical, which may contain at least one nitrogen atom as ring member and/or which may be at least mono-substituted by a linear or branched C₁₋₄ alkyl radical, or a phenyl radical, preferably a hydrogen atom, a linear or branched C₁₋₄ alkyl radical, a vinyl group, a cyclohexyl radical, an N-Methyl-piperidyl radical or a phenyl radical.

In a preferred aspect of the invention the compound according to formula II used is characterized in that R³² represents a hydrogen atom, an optionally at least one nitrogen atom as ring member containing, 5- or 6-membered cycloaliphatic radical, which may be at least mono-substituted by a linear or branched C₁₋₄-alkyl radical and/or which may be bound via a linear or branched C₁₋₄-alkylene group, a NR³³R³⁴⁻moiety, which is bound via a linear or branched C₁₋₄ alkylene group, or a NR³⁵R³⁶⁻moiety, which is bound via a linear or branched C₁₋₄ alkylene group, preferably a hydrogen atom, an optionally at least one nitrogen atom as ring member containing, 5- or 6-membered cycloaliphatic radical, which may be at least mono-substituted by a linear or branched C₁₋₄-alkyl radical and/or which may be bound via a linear or branched C₁₋₄-alkylene group, a NR³³R³⁴-moiety, which is bound via a linear or branched C₂₋₃ alkylene group, or a NR³⁵R³⁶-moiety, which is bound via a linear or branched C₂₋₃ alkylene group.

In a preferred aspect of the invention the compound according to formula II used is characterized in that R³³ and R³⁴, identical or different, independently from one another represent hydrogen; a linear or branched C₁₋₄ alkyl radical or an unsubstituted benzyl radical, preferably hydrogen or a linear or branched C₁₋₄ alkyl radical.

In a preferred aspect of the invention the compound according to formula II used is characterized in that R³⁵ and R³⁶ together with the bridging nitrogen atom represent a saturated, unsubstituted, optionally at least one oxygen atom as ring member containing, 5- or 6-membered heterocyclic radical.

In a preferred aspect of the invention the compound according to formula II used is characterized in that X represents an optionally at least mono-substituted phenyl radical or an optionally at least mono-substituted thienyl radical, wherein in each case the substituents may be independently selected from the group consisting of a linear or branched C₁₋₄ alkyl radical, a linear or branched C₁₋₄ alkoxy radical, a linear or branched C₁₋₄ alkyl radical, which is at least partially fluorinated, a fluorine atom, a chlorine atom and a bromine atom, preferably represents an optionally at least mono-substituted phenyl radical or an optionally at least mono-substituted thienyl radical, wherein in each case the substituents may be independently selected from the group consisting of a methyl radical, a methoxy radical, a trifluoromethyl radical, a fluorine atom, a chlorine atom and a bromine atom.

In a preferred aspect of the invention the compound according to formula II used is characterized in that Y represents an azole radical selected from the group consisting of
a) a pyrazole of the general formula (a): 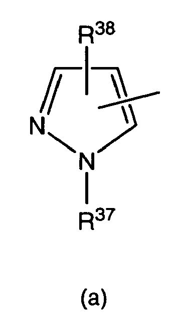 in which R³⁷ represents a linear or branched C₁-₁₂ alkyl radical, a benzyl radical or a radical of the type: 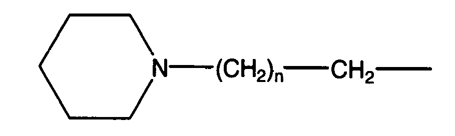 in which n = 1 or 2, and
   R³⁸ represents a hydrogen atom, a methyl radical or a halogen atom, preferably a hydrogen atom, a methyl radical, a bromine atom or a chlorine atom,
b) an imidazole of the general formula 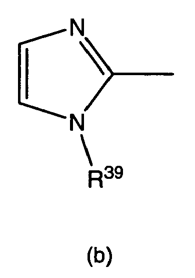 in which R³⁹ represents a hydrogen atom, a C₁-₁₂ alkyl radical, a benzyl radical, or a radical of the general formula (b1):

   R⁴⁰-(CH₂)ₙ- (b1)

   in which n is 2, 3 or 4 and R⁴⁰ represents a piperidinyl radical, a phenyl radical, a cyano group, a hydroxyl radical, a carboxy radical, an amino group, a dimethylamino group, or a methyl ester (CH₃-O-C(=O)-) group,
   and
(c) an imidazole of the following formula:

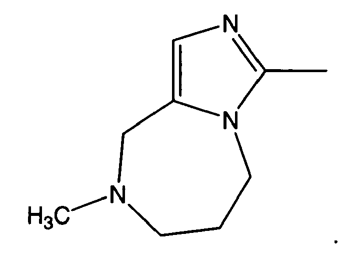

In a preferred aspect of the invention at least one compound used according to the invention is a carbinol compound of general formula II 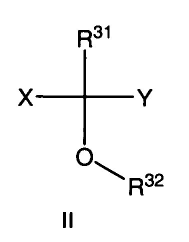 is present, wherein
R³¹ represents a hydrogen atom, a methyl radical, an ethyl radical, an n-propyl radical, an iso-propyl radical, a sec-butyl radical, a tert-butyl radical, an n-butyl radical, a vinyl radical, a cyclohexyl radical, an N-methyl-piperidinyl group, or a phenyl group,
R³² represents a hydrogen atom, a monomethylaminoethyl group, a dimethylaminoethyl group, an aminoethyl group, a pyrrolidinylethyl group, a piperidinylethyl group, a methyl-benzyl-aminoethyl group, a morpholinylethyl group, a diisopropylaminoethyl group, a dimethylaminopropyl group, a piperidinylpropyl group, a pyrrolidinylpropyl group, a morpholinylpropyl group, an N-methyl-2-piperidyl group, an N-ethyl-2-piperidyl group, an N-propyl-2-piperidyl group, an N-methyl-2-pyrrolidinyl group, an N-ethyl-2-pyrrolidinyl group, an N-propyl-2-pyrrolidinyl group, or a 2-dimethylaminoethyl-1-methyl group,
X represents a phenyl radical, a 2-methyl-phenyl radical, a 3-methyl-phenyl radical, a 4-methyl phenyl radical, a 2-chloro-phenyl radical, a 3-chloro-phenyl radical, a 4-chloro-phenyl radical, a 2-fluoro-phenyl radical, a 3-fluoro-phenyl radical, a 4-fluoro-phenyl radical, a 2-trifluoromethyl-phenyl radical, a 3-trifluoromethyl-phenyl radical, a 4-trifluoromethyl-phenyl radical, a 2-methoxy-phenyl radical, a 3-methoxy-phenyl radical, a 4-methoxy-phenyl radical, a 3,4,5-tris-methoxy phenyl radical, a 3,4-dichloro-phenyl radical, a 2,4-dichlorophenylradical, a thien-2-yl radical, a thien-3-yl radical, a 3-methyl-thien-2-yl radical, a 5-methyl-thien-2-yl-radical, a 5-bromo-thien-2-yl radical or a 4-bromothien-2-yl-radical,
Y represents an azole radical selected from the group consisting of
a) a pyrazole of the general formula (a): 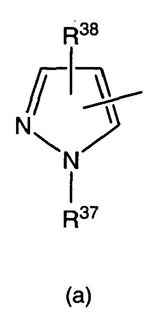 in which
   R³⁷ represents a methyl radical, an ethyl radical, an n-propyl radical, an isopropyl radical, an n-butyl radical, a sec-butyl radical or a tert-butyl radical,
   R³⁸ represents a hydrogen atom, a methyl radical, a bromine atom or a chlorine atom,
b) an imidazole of the general formula 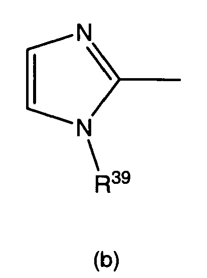 in which R³⁹ represents a hydrogen atom, a methyl radical, an ethyl radical, an n-propyl radical, an iso-butyl radical, an n-butyl radical, a sec-butyl radical a tert-butyl radical, an n-pentyl radical, an n-hexyl radical, an n-heptyl radical, an n-octyl radical, an n-nonyl radical, an n-decyl radical, an n-undecyl radical an n-dodecyl radical, a benzyl radical, or a radical of the general formula (b1):

   R⁴⁰-(CH₂)ₙ (b1)

   in which n is 2, 3 or 4 and R⁴⁰ represents a piperidinyl radical, a phenyl radical, a cyano group, a hydroxyl radical, a carboxy radical, an amino group, a dimethylamino group, or a methyl ester group,
   and
(c) an imidazole of the following formula: 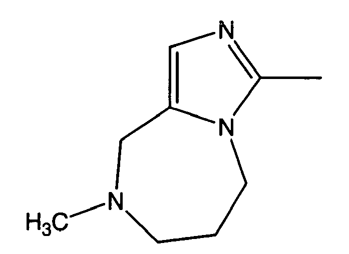 optionally in form of one of its stereoisomers, preferably enantiomers or diastereomers, its racemate or in form of a mixture of at least two of its stereoisomers, preferably enantiomers and/or diastereomers, in any mixing ratio, or a corresponding salt thereof, or a corresponding solvate.

In a preferred aspect of the invention at least one compound according to formula II used is selected from the group consisting of
[1] 2-{α-[2-(dimethylamino)ethoxy]benzyl}-1-methyl-1*H*-imidazole,
[2] 2-{4-chloro-α-[2-(dimethylamino)ethoxy]-α-methylbenzyl}-1-methyl-1*H*- imidazole,
[3] 2-{4-chloro-α-[2-(dimethylamino)ethoxy]benzyl}-1-methyl-1*H-*imidazole,
[4] 2-{3-chloro-α-[2-(dimethylamino)ethoxy]benzyl}-1-methyl-1*H-*imidazole,
[5] 2-{4-chloro-α-[2-(dimethylamino)ethoxy]-α-methylbenzyl}-1-methyl-1*H*- imidazole,
[6] 2-{4-fluoro-α-[2-(dimethylamino)ethoxy]-α-methylbenzyl}-1-methyl-1*H*- imidazole,
[7] 2-{α-[2-(dimethylamino)ethoxy]-α-methyl-3-(trifluoromethyl)benzyl}-1-methyl- 1*H*-imidazole,
[8] 2-{3-chloro-α-[2-(dimethylamino)ethoxy]-α-methylbenzyl}-1-methyl-1*H*- imidazole,
[9] 2-{3-chloro-α-[2-(dimethylamino)ethoxy]-α-propylbenzyl}-1-methyl-1*H*- imidazole,
[10] 1-butyl-2-{4-chloro-α-[2-(dimethylamino)ethoxy]-α-methylbenzyl}-1*H*-imidazole,
[11] 2-{α-[2-(dimethylamino)ethoxy]-α-methyl-4-methoxybenzyl}-1-methyl-1*H*-imidazole,
[12] 2-{3-chloro-α-methyl-α-[2-(N-pyrrolidinyl)ethoxy]benzyl}-1-methyl-1*H*-imidazole,
[13] 2-{α-[2-(dimethylamino)ethoxy]-α-propyl-3,4,5-trimethoxybenzyl}-1-dodecyl-1*H*-imidazole,
[14] 1-butyl-2-{α-[2-(dimethylamino)ethoxy]-4-(trifluoromethyl)benzyl}-1*H*-imidazole,
[15] 1-methyl-2-{α-methyl-α-[2-(N-piperidyl)ethoxy]-3-(trifluoromethyl)benzyl}-1*H*-imidazole,
[16] 2-{α-cyclohexyl-3,4-dichloro-α-[2-(dimethylamino)ethoxy]benzyl}-1-methyl-1*H*-imidazole,
[17] 2-{3,4-dichloro-α-[2-(dimethylamino)ethoxy]-α-propylbenzyl}-1-methyl-1*H*-imidazole,
[18] 2-{3,4-dichloro-α-[2-(dimethylamino)ethoxy]-α-methylbenzyl}-1-methyl-1*H*-imidazole,
[19] 2-{3,4-dichloro-α-[2-(dimethylamino)ethoxy]benzyl}-1-methyl-1*H*-imidazole,
[20] 2-{4-chloro-α-[2-(dimethylamino)ethoxy]-α-methylbenzyl}-1-[2-(N-piperidyl)ethyl]-1*H*-imidazole,
[21] 2-{4-chloro-α-[2-(dimethylamino)ethoxy]-α-methylbenzyl}-1-[2-(N-piperidyl)propyl]-1*H*-imidazole,
[22] 1-(3-cyanopropyl)-2-{4-chloro-α-[2-(dimethylamino)ethoxy]benzyl}-1*H*-imidazole,
[23] 2-{4-chloro-α-[2-(dimethylamino)ethoxy]-α-(N-methyl-4-piperidyl)benzyl}-1-methyl-1*H*-imidazole,
[24] 1-benzyl-2-{α-[2-(N-benzyl-N-methylamino)ethoxy]-4-chlorobenzyl}-1*H*-imidazole,
[25] 2-{4-chloro-α-[2-(dimethylamino)ethoxy]-α-methylbenzyl}-7-methyl-6,7,8,9-tetrahydro-1*H*-imidazole[1,5-a][1,4]diazepine,
[26] 2-{4-chloro-α-[2-(dimethylamino)ethoxy]benzyl}-7-methyl-6,7,8,9-tetrahydro-1*H*-imidazole[1,5-a][1,4]diazepine,
[27] 1-butyl-5-{α-[2-(dimethylamino)ethoxy]benzyl}-1*H*-pyrazole,
[28] 5-{α-(4-chlorophenyl)-α-[2-(dimethylamino)ethoxy]benzyl}-1-methyl-1*H*-pyrazole,
[29] 1-butyl-5-{α-[2-(dimethylamino)ethoxy]-3,4,5-trimethoxybenzyl}-1*H*-pyrazole,
[30] 1-butyl-5-{4-chloro-α-[2-(dimethylamino)ethoxy]-α-methylbenzyl}-1*H*-pyrazole,
[31] 5-{α-[2-(dimethylamino)ethoxy]benzyl}-1-methyl-1*H*-pyrazole,
[32] 5-{α-[2-(dimethylamino)ethoxy]-α-methylbenzyl}-1-methyl-1*H*-pyrazole,
[33] 5-{α-[2-(dimethylamino)ethoxy]-3,4,5-trimethoxybenzyl}-1-methyl-1*H*-pyrazole,
[34] 1-methyl-5-{α-[2-(N-pyrrolidinyl)ethoxy]benzyl}-1*H*-pyrazole,
[35] 1-methyl-5-{α-[2-(N-morpholinyl)ethoxy]benzyl}-1*H*-pyrazole,
[36] 5-{α-[2-(dimethylamino)ethoxy]-α-methyl-3,4,5-trimethoxybenzyl}-1-methyl-1*H*-pyrazole,
[37] 4-bromo-5-{α-[2-(dimethylamino)ethoxy]benzyl}-1-methyl-1*H-*pyrazole,
[38] 1,3-dimethyl-5-{α-[2-(dimethylamino)ethoxy]-α-methylbenzyl}-1*H*-pyrazole,
[39] 1,3-dimethyl-5-{α-[2-(dimethylamino)ethoxy]benzyl}-1*H-*pyrazole,
[40] 5-{α-[2-(dimethylamino)ethoxy]-2-methylbenzyl}-1-methyl-1*H-*pyrazole,
[41] 4-chloro-5-{4-chloro-α-[2-(dimethylamino)ethoxy]benzyl}-1-methyl-1*H*-pyrazole,
[42] 5-{4-chloro-α-[2-(dimethylamino)ethoxy]benzyl}-1-methyl-1*H-*pyrazole,
[43] 5-{3-chloro-α-[2-(dimethylamino)ethoxy]benzyl}-1-methyl-1*H-*pyrazole,
[44] 5-{α-[2-(dimethylamino)ethoxy]-4-methylbenzyl}-1-methyl-1*H-*pyrazole,
[45] 5-{2-chloro-α-[2-(dimethylamino)ethoxy]benzyl}-1-methyl-1*H-*pyrazole,
[46] 1-methyl-5-{α-[2-(N-piperidyl)ethoxy]benzyl}-1*H*-pyrazole,
[47] 1-methyl-5-{α-[2-(N-propyl-2-piperidyl)ethoxy]benzyl}-1*H-*pyrazole,
[48] 5-{α-[2-(N-ethyl-2-piperidyl)ethoxy]benzyl}-1-methyl-1*H-*pyrazole,
[49] 1-methyl-5-{α-[2-(N-methyl-2-pyrrolidinyl)ethoxy]benzyl}-1*H-*pyrazole,
[50] 5-{α-[2-(diisopropylamino)ethoxy]benzyl}-1-methyl-1*H-*pyrazole,
[51] 1-methyl-5-{α-[2-(N-methyl-2-piperidyl)ethoxy]benzyl}-1*H-*pyrazole,
[52] 2-{4-chloro-α-[3-(dimethylamino)propoxy]-α-methylbenzyl}-1-methyl-1*H*-imidazole,
[53] 2-{3-chloro-α-[3-(dimethylamino)propoxy]benzyl}-1-methyl-1*H-*imidazole,
[54] 2-{4-chloro-α-[3-(dimethylamino)propoxy]-α-ethylbenzyl}-1-methyl-1*H*-imidazole,
[55] 2-{α-butyl-3-chloro-α-[3-(dimethylamino)propoxy]benzyl}-1-methyl-1*H*-imidazole,
[56] 2-{α-cyclohexyl-4-chloro-α-[3-(dimethylamino)propoxy]benzyl}-1-methyl-1*H*-imidazole,
[57] 2-{α-[3-(dimethylamino)propoxy]-4-fluoro-α-methylbenzyl}-1-methyl-1*H*-imidazole,
[58] 2-{α-[3-(dimethylamino)propoxy]-α-methyl-3-(trifluoromethyl)benzyl}-1-methyl-1*H*-imidazole,
[59] 2-{2-chloro-α-[3-(dimethylamino)propoxy]-α-methylbenzyl}-1-methyl-1*H*-imidazole,
[60] 2-{3-chloro-α-[3-(dimethylamino)propoxy]-α-methylbenzyl}-1-methyl-1*H*-imidazole,
[61] 2-{α-[3-(dimethylamino)propoxy]-α-methyl-3,4,5-trimethoxybenzyl}-1-methyl-1*H*-imidazole,
[62] 2-{α-[3-(dimethylamino)propoxy]-α-methyl-4-methoxybenzyl}-1-methyl-1*H*-imidazole,
[63] 2-{4-chloro-α-[3-(dimethylamino)propoxy]benzyl}-1-methyl-1*H-*imidazole,
[64] 2-{α-[3-(dimethylamino)propoxy]-3,4,5-trimethoxybenzyl}-1-methyl-1*H*-imidazole,
[65] 2-{α-[3-(dimethylamino)propoxy]-α-methyl-4-(trifluoromethyl)benzyl}-1-methyl-1*H*-imidazole,
[66] 2-{α-[3-(dimethylamino)propoxy]-3-(trifluoromethyl)benzyl}-1-methyl-1*H*-imidazole,
[67] 2-{α-[3-(dimethylamino)propoxy]-4-(trifluoromethyl)benzyl}-1-methyl-1*H*-imidazole,
[68] 2-{α-[3-(dimethylamino)propoxy]-4-methoxybenzyl}-1-methyl-1*H*-imidazole,
[69] 2-{α-butyl-α-[3-(dimethylamino)propoxy]-3-(trifluoromethyl)benzyl}-1-methyl-1*H*-imidazole,
[70] 1-butyl-2-{4-chloro-α-[3-(dimethylamino)propoxy]-α-methylbenzyl}-1*H*-imidazole,
[71] 1-butyl-2-{α-butyl-α-[3-(dimethylamino)propoxy]-3,4,5-trimethoxybenzyl}-1*H*-imidazole,
[72] 1-butyl-2-{α-butyl-2-chloro-α-[3-(dimethylamino)propoxy]benzyl}-1*H*-imidazole,
[73] 1-butyl-2-{α-butyl-2,4-dichloro-α-[3-(dimethylamino)propoxy]benzyl}-1*H*-imidazole,
[74] 1-butyl-2-{α-[3-(dimethylamino)propoxy]-4-(trifluoromethyl)benzyl}-1*H*-imidazole,
[75] 2-{4-chloro-α-[3-(N-piperidyl)propoxy]benzyl}-1-methyl-1*H-*imidazole,
[76] 1-methyl-2-{α-methyl-α-[3-(N-piperidyl)propoxy]-4-(trifluoromethyl)benzyl}-1*H*-imidazole,
[77] 2-{α-butyl-2-chloro-α-[3-(dimethylamino)propoxy]benzyl}-1-methyl-1*H*-imidazole,
[78] 2-{α-butyl-3,4-dichloro-α-[3-(dimethylamino)propoxy]benzyl}-1-methyl-1*H*-imidazole,
[79] 2-{3,4-dichloro-α-[3-(dimethylamino)propoxy]-α-methylbenzyl}-1-methyl-1*H*-imidazole,
[80] 2-{3,4-dichloro-α-[3-(dimethylamino)propoxy]benzyl}-1-methyl-1*H*-imidazole,
[81] 2-{α-cyclohexyl-3,4-dichloro-α-[3-(dimethylamino)propoxy]benzyl}-1-methyl-1*H*-imidazole,
[82] 2-{4-chloro-α-[3-(dimethylamino)propoxy]-α-methylbenzyl}-α-[2-(N-piperidyl) ethyl]-1*H*-imidazole,
[83] 2-{4-chloro-α-[3-(dimethylamino)propoxy]-α-methylbenzyl}-1-[2-(N-piperidyl) propyl]-1*H*-imidazole,
[84] 2-{4-chloro-α-[3-(dimethylamino)propoxy]-α-(N-methyl-4-piperidyl)benzyl}-1-methyl-1*H*-imidazole,
[85] 1-butyl-5-{α-[3-(dimethylamino)propoxy]benzyl}-1*H*-pyrazole,
[86] 1-butyl-5-{4-chloro-α-[3-(dimethylamino)propoxy]-α-methylbenzyl}-1*H*- pyrazole,
[87] 5-{α-[3-(dimethylamino)propoxy]benzyl}-1-methyl-1*H-*pyrazole,
[88] 5-{α-[3-(dimethylamino)propoxy]-α-methylbenzyl}-1-methyl-1*H*-pyrazole,
[89] 1,3-dimethyl-5-{α-[3-(dimethylamino)propoxy]-α-methylbenzyl}-1*H*-pyrazole,
[90] 1,3-dimethyl-5-{α-[3-(dimethylamino)propoxy]benzy)}-1*H-*pyrazole,
[91] 5-{α-[3-(dimethylamino)propoxy]-2-methylbenzyl}-1-methyl-1*H*-pyrazole,
[92] 5-chloro-5-{4-chloro-α-[3-(dimethylamino)propoxy]benzyl}-1-methyl-1*H*-pyrazole,
[93] 1-methyl-5-{α-[3-(N-piperidyl)propoxy]benzyl}-1*H*-pyrazole,
[94] 1-methyl-5-{α-[3-(N-pyrrolidinyl)propoxy]benzyl}-1*H*-pyrazole,
[95] 4-{4-chloro-α-[2-(dimethylamino)ethoxy]benzyl}-1-methyl-1*H-*pyrazole,
[96] 4-{4-chloro-α-[2-(dimethylamino)ethoxy]-α-methylbenzyl}-1-methyl-1*H*-pyrazole,
[97] 4-{4-chloro-α-[2-(N-propyl-2-piperidyl)ethoxy]benzyl}-1-methyl-1*H*-pyrazole,
[98] 4-{4-chloro-α-[2-(N-methyl-2-piperidyl)ethoxy]benzyl}-1-methyl-1*H*-pyrazole,
[99] 4-{4-chloro-α-[2-(N-ethyl-2-piperidyl)ethoxy]benzyl}-1-methyl-1*H*-pyrazole,
[100] 4-{4-chloro-α-[2-(diisopropylamino)ethoxy]benzyl}-1-methyl-1*H*-pyrazole,
[101] 4-{4-chloro-α-[2-(N-methyl-2-pyrrolidinyl)ethoxy]benzyl}-1-methyl-1*H*-pyrazole,
[102] 4-{α-[3-(dimethylamino)propoxy]benzyl}-1-methyl-1*H-*pyrazole,
[103] 4-{4-chloro-α-[3-(N-morpholinyl)propoxy]benzyl}-1-methyl-1*H-*pyrazole,
[104] 4-{4-chloro-α-[3-(N-pyrrolidinyl)propoxy]benzyl}-1-methyl-1*H-*pyrazole,
[105] 2-(α-hydroxybenzyl)-1*H*-imidazole,
[106] 2-(4-chloro-α-hydroxybenzyl)-1*H*-imidazole,
[107] 2-(4-chloro-α-hydroxybenzyl)-1-methyl-1*H*-imidazole,
[108] 2-(3-chloro-α-hydroxybenzyl)-1-methyl-1*H*-imidazole,
[109] 2-(4-fluoro-α-hydroxybenzyl)-1-methyl-1*H*-imidazole,
[110] 2-[α-hydroxy-3-(trifluoromethyl)benryl]-1-methyl-1*H*-imidazole,
[111] 2-[α-hydroxy-4-(trifluoromethyl)benzyl]-1-methyl-1*H*-imidazole,
[112] 2-(α-hydroxy-3,4,5-trimethoxybenzyl)-1-methyl-1*H*-imidazole,
[113] 2-(3,4-dichloro-α-hydroxybenzyl)-1-methyl-1*H*-imidazole,
[114] 1-butyl-2-[α-hydroxy-4-(trifluoromethyl)benzyl]-1*H*-imidazole,
[115] 1-butyl-2-(3,4-dichloro-α-hydroxybenzyl)-1*H*-imidazole,
[116] 1-butyl-2-(4-chloro-α-hydroxybenzyl)-1*H*-imidazole,
[117] 1-butyl-2-(α-hydroxy-3,4,5-trimethoxybenzyl)-1*H*-imidazole,
[118] 1-dodecyl-2-(α-hydroxy-3,4,5-trimethoxybenzyl)-1*H*-imidazole,
[119] 2-(α-butyl-3-chloro-α-hydroxybenzyl)-1-methyl-1*H*-imidazole,
[120] 2-(3-chloro-α-hydroxy-α-methylbenzyl)-1-methyl-1*H*-imidazole,
[121] 2-(4-chloro-α-hydroxy-α-methylbenzyl)-1-methyl-1*H-*imidazole,
[122] 2-[4-chloro-α-hydroxy-α-(N-methyl-4-piperidyl)benzyl]-1-methyl-1*H*-imidazole,
[123] 2-(4-chloro-α-ethyl-α-hydroxybenzyl)-1-methyl-1*H*-imidazole,
[124] 2-(α-butyl-4-chloro-α-hydroxybenzyl)-1-methyl-1*H*-imidazole,
[125] 2-(α-cyclohexyl-4-chloro-α-hydroxybenzyl)-1-methyl-1*H-*imidazole,
[126] 2-(2-chloro-α-hydroxy-α-methylbenzyl)-1-methyl-1*H*-imidazole,
[127] 2-(α-butyl-2-chloro-α-hydroxybenzyl)-1-methyl-1*H*-imidazole,
[128] 2-[α-hydroxy-α-methyl-3-(trifluoromethyl)benzyl]-1-methyl-1*H*-imidazole,
[129] 2-[α-butyl-α-hydroxy-3-(trifluoromethyl)benzyl]-1-methyl-1*H-*imidazole,
[130] 2-[α-cyclohexyl-α-hydroxy-3-(trifluoromethyl)benzyl]-1-methyl-1*H*-imidazole,
[131] 2-[α-hydroxy-α-methyl-4-(trifluoromethyl)benzyl]-1-methyl-1*H-*imidazole,
[132] 2-(4-fluoro-α-hydroxy-α-methylbenzyl)-1-methyl-1*H*-imidazole,
[133] 2-(α-hydroxy-α-methyl-4-methoxybenzyl)-1-methyl-1*H-*imidazole,
[134] 2-(3,4-dichloro-α-hydroxy-α-methylbenzyl)-1-methyl-1*H-*imidazole,
[135] 2-(α-butyl-3,4-dichloro-α-hydroxybenzyl)-1-methyl-1*H-*imidazole,
[136] 2-(α-cyclohexyl-3,4-dichloro-α-hydroxybenzyl)-1-methyl-1*H-*imidazole,
[137] 2-(α -hydroxy-α-methyl-3,4,5-trimethoxybenzyl)-1-methyl-1*H-*imidazole,
[138] 1-butyl-2-(4-chloro-α-hydroxy-α-methylbenzyl)-1*H*-imidazole,
[139] 1-butyl-2-(α-butyl-4-chloro-α-hydroxybenzyl]-1*H*-imidazole,
[140] 1-butyl-2-[4-chloro-α-hydroxy-α-(N-methyl-4-piperidyl)benzyl]-1*H*-imidazole,
[141] 1-butyl-2-(α-butyl-α-hydroxy-3,4,5-trimethoxybenzyl)-1*H-*imidazole,
[142] 1-butyl-2-(α-butyl-2-chloro-α-hydroxybenzyl)-1*H*-imidazole,
[143] 1-butyl-2-[α-ethyl-α-hydroxy-3-(trifluoromethyl)benzyl]-1*H-*imidazole,
[144] 1-butyl-2-(α-butyl-2,4-dichloro-α-hydroxybenzyl)-1*H*-imidazole,
[145] 2-(4-chloro-α-hydroxy-α-methylbenzyl)-1-[2-(N-piperidyl)ethyl]-1*H*-imidazole,
[146] 2-(4-chloro-α-hydroxy-α-methylbenzyl)-1-(3-dimethylaminopropyl)-1 *H*-imidazole,
[147] 2-(α-butyl-α -hydroxy-3,4,5-trimethoxybenzyl)-1-dodecyl-1*H-*imidazole,
[148] 1-benzyl-2-[α-butyl-α-hydroxy-3-(trifluoromethyl)benzyl]-1*H-*imidazole,
[149] 1-benzyl-2-(4-chloro-α-hydroxy-α-methylbenzyl)-1*H*-imidazole,
[150] 1-(2-cyanoethyl)-2-(4-chloro-α-hydroxybenzyl)-1*H*-imidazole,
[151] 1-(3-aminopropyl)-2-(4-chloro-α-hydroxybenzyl)-1*H*-imidazole,
[152] 3-[2-(3-chloro-α-hydroxybenzyl)-1*H*-imidazole-1-yl]propanoic acid,
[153] 2-(4-chloro-α-hydroxybenzyl)-1-(3-hydroxypropyl)-1*H-*imidazole,
[154] 3-[2-(3-chloro-α-hydroxybenzyl)-1*H*-imidazole-1-yl]methylpropanoate,
[155] 2-(α-hydroxybenzyl)-1-(3-hydroxypropyl)-1*H*-imidazole,
[156] 2-(α-hydroxy-4-methylbenzyl)-1-(3-hydroxypropyl)-1*H-*imidazole,
[157] 2-(α-hydroxy-4-methoxybenzyl)-1-(3-hydroxypropyl)-1*H-*imidazole,
[158] 2-(3,4-dichloro-α-hydroxybenzyl)-1-(3-hydroxypropyl)-1*H-*imidazole,
[159] 3-{2-(α-hydroxybenzyl)-1*H*-imidazole-1-yll}-methyl propanoate,
[160] 2-(4-chloro-α-hydroxybenzyl)-1-(4-hydroxybutyl)-1*H*-imidazole,
[161] 1-(3-cyanopropyl)-2-(4-chloro-α-hydroxybenzyl)-1*H*-imidazole,
[162] 4-[2-(4-chloro-α-hydroxybenzyl)-1*H*-imidazole-1-yl]butanoic acid,
[163] 4-[2-(4-chloro-α-hydroxybenzyl)-1*H*-imidazole-1-yl]-methyl butanoate,
[164] 1-butyl-5-(α-hydroxybenzyl)-1*H*-pyrazole,
[165] 5-(4-chloro-α-hydroxybenzyl)-1-methyl-1*H*-pyrazole,
[166] 5-(α-hydroxy-3,4,5-trimethoxybenzyl)-1-methyl-1*H*-pyrazole,
[167] 1-butyl-5-(α-hydroxy-3,4,5-trimethoxybenzyl)-1*H*-pyrazole,
[168] 4-bromo-5-(α-hydroxybenzyl)-1-methyl-1*H*-pyrazole,
[169] 5-[α-(4-chlorophenyl)-α-hydroxybenzyl]-1-methyl-1*H*-pyrazole,
[170] 1-butyl-5-(4-chloro-α-hydroxy-α-methylbenzyl)-1*H*-pyrazole,
[171] 5-(α-hydroxy-α-methylbenzyl)-1-methyl-1*H*-pyrazole,
[172] 5-(α-hydroxy-α-methyl-3,4,5-trimethoxybenzyl)-1-methyl-1*H-*pyrazole,
[173] 1,3-dimethyl-5-(α-hydroxy-α-methylbenzyl)-1*H*-pyrazole,
[174] 1-butyl-5-(α-hydroxy-α-vinylbenzyl)-1*H*-pyrazole,
[175] 1-butyl-5-(4-chloro-α-hydroxy-α-vinylbenzyl)-1*H*-pyrazole,
[176] 4-chloro-5-(α-hydroxybenzyl)-1-methyl-1*H*-pyrazole,
[177] 5-(α-hydroxy-2-methylbenzyl)-1-methyl-1*H*-pyrazole,
[178] 5-(3-chloro-α-hydroxybenzyl)-1-methyl-1*H*-pyrazole,
[179] 5-(α-hydroxy-4-methylbenzyl)-1-methyl-1*H*-pyrazole,
[180] 5-(2-chloro-α-hydroxybenzyl)-1-methyl-1*H*-pyrazole,
[181] 5-(α-hydroxy-4-methoxybenzyl)-1-methyl-1*H*-pyrazole,
[182] 5-{α-[2-(dimethylamino)ethoxy]-2-thienylmethyl}-2-methyl-1*H-*pyrazole,
[183] 5-{α-[2-(dimethylamino)ethoxy]-2-thienylmethyl}-1-methyl-1*H-*pyrazole citrate,
[184] 5-{α-[2-(dimethylamino)ethoxy]-3-thienylmethyl}-1-methyl-1*H-*pyrazole,
[185] 2-{α-[2-(dimethylamino)ethoxyl-2-thienylmethyl}-1-methyl-1 *H-*imidazole,
[186] 5-{α-[2-(dimethylamino)ethoxyl-3-methyl-2-thienylmethyl}-1-methyl-1*H*-pyrazole,
[187] 5-{α-[2-(dimethylamino)ethoxy]-5-methyl-2-thienylmethyl}-1-methyl-1*H*-pyrazole,
[188] 5-{5-bromo-α-[2-(dimethylamino)ethoxy]-2-thienylmethyl}-1-methyl-1*H*-pyrazole,
[189] 5-{4-bromo-α-[2-(dimethylamino)ethoxy]-2-thienylmethyl}-1-methyl-1*H*-pyrazole,
[190] 5-{α-[2-(dimethylamino)ethoxy]-α-methyl-2-thienylmethyl}-1-methyl-1*H*-pyrazole,
[191] 5-{α-[2-(dimethylamino)ethoxy]benzyl}-1-methyl-1*H*-pyrazole citrate,
[192] (±)-5-{α-[2-(dimethylamino)-1-(methyl)ethoxy]benzyl}-1-methyl-1*H*-pyrazole,
[193] (±)-5-{α-[2-(dimethylamino)-1-(methyl)ethoxy]benzyl)-1-methyl-1*H*-pyrazole,
[194] (+)-5-{α-[2-(dimethylamino)ethoxy]-2-thienylmethyl}-1-methyl-1*H*-pyrazole,
[195] (-)-5-{α-[2-(dimethylamino)ethoxy]-2-thienylmethyl}-1-methyl-1*H*-pyrazole,
[196] (+)-5-{α-[2-(dimethylamino)ethoxy]-2-thienylmethyl}-1-methyl-1*H*-pyrazole citrate,
[197] (-)-5-{α-[2-(dimethylamino)ethoxy]-2-thienylmethyl}-1-methyl-1*H*-pyrazole citrate,
[198] (+)-5-{α-[2-(dimethylamino)ethoxy]-2-thienylmethyl}-1-methyl-1*H*-pyrazole-D-ditoluyltartrat,
[199] (-)-5-{α-[2-(dimethylamino)ethoxy]-2-thienylmethyl}-1-methyl-1*H*-pyrazole D-ditoluyltartrat,
[200] (+)-5-{α-[2-(dimethylamino)ethoxy]benzyl}-1-methyl-1*H-*pyrazole citrate,
[201] (-)-5-{α-[2-(dimethylamino)ethoxy]benzyl}-1-methyl-1*H-*pyrazole citrate,
[202] 5-(α-hydroxy-2-thienylmethyl)-1-methyl-1*H*-pyrazole,
[203] 5-(α-hydroxy-3-methyl-2-thienylmethyl)-1-methyl-1*H*-pyrazole,
[204] 5-(α-hydroxy-5-methyl-2-thienylmethyl)-1-methyl-1*H*-pyrazole,
[205] 5-(5-bromo-α-hydroxy-2-thienylmethyl)-1-methyl-1*H*-pyrazole,
[206] 5-(4-bromo-α-hydroxy-2-thienylmethyl)-1-methyl-1*H*-pyrazole,
[207] 5-(α-hydroxy-α-methyl-2-thienylmethyl)-1-methyl-1*H*-pyrazole,
[208] 2-((1-methyl-1H-pyrazol-5-yl)(thiophen-2-yl)methoxy)ethanamine,
[209] 2-((1-methyl-1H-pyrazol-5-yl)(thiophen-2-yl)methoxy)-N-methylethanamine,
[210] 2-((1-methyl-1H-pyrazol-5-yl)(phenyl)methoxy)ethanamine
   and
[211] 2-((1-methyl-1H-pyrazol-5-yl)(phenyl)methoxy)-N-methylethanamine.

In a highly preferred embodiment of the invention the compound used (for the manifacture of a medicament for the treatment of neuropathic pain) is of the general formula (I) 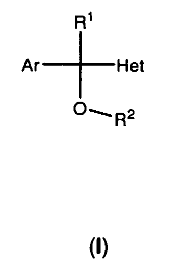 in which
Ar represents a phenyl radical or a thienyl radical, with no substitutions or optionally with 1, 2 or 3 equal or different substituents, selected from a group consisting of fluoride, chloride, bromide, methyl, trifluoromethyl and methoxy;
R¹ represents hydrogen or a lower alkyl group from C₁ to C₄;
R² represents a dialkyl(C₁-C₄)aminoalkyl (C₂-C₃), a monoalkyl(C₁-C₄)aminoalkyl (C₂-C₃), an aminoalkyl (C₂-C₃), or azaheterocyclylalkyl (C₂-C₃) radical; and
Het represents a five-armed nitrogenated aromatic heterocycle that contains one to three nitrogen atoms, without substitutions or optionally substituted by 1 or 2 equal or different substituents selected from a group consisting of fluoride, chloride, bromide and methyl;
optionally in the form of its racemate, pure stereoisomers, especially enantiomers or diastereomers or in the form of mixtures of stereoisomers, especially enantiomers or diastereomers, in any suitable ratio;
in the form shown or in form of the acid or base or in form of a salt, especially a physiologically acceptable salt, or in form of a solvate, especially a hydrate.

In a preferred aspect of the invention the compound according to formula I used is characterized in that R¹ is selected from hydrogen or from a group consisting of methyl, ethyl, propyl, isopropyl, butyl, isobutyl, *sec*-butyl and *tert*-butyl.

In a preferred aspect of the invention the compound according to formula I used is characterized in that R² is selected from among a group consisting of dimethylaminoethyl, dimethylaminopropyl, diethylaminoethyl, diethylaminopropyl, methylaminoethyl, methylaminopropyl, aminoethyl, aminopropyl, piperidinylethyl, piperidinylpropyl, morpholinylpropyl, morpholinylethyl, pirrolidinylpropyl and pirrolidinylethyl;
preferably
dimethylaminoethyl, dimethylaminopropyl, diethylaminoethyl, piperidinylethyl, morpholinylpropyl, and pirrolidinylethyl.

In a preferred aspect of the invention the compound according to formula I used is a compound of general formula (Ia) 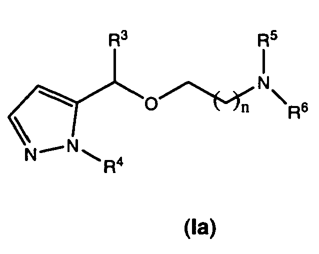
in which
n is 1 or 2;
R³ is selected from: 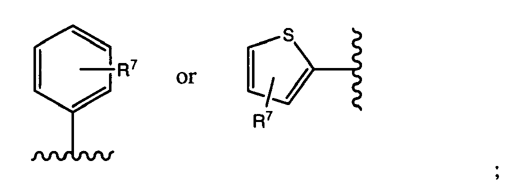
R⁴ is selected from hydrogen, fluoride, chloride, bromide and methyl;
R⁵ and R⁶ are independently selected from hydrogen, lower C₍₁₋₄₎-Alkyl or together with the Nitrogen form an azaheterocyclic ring;
R⁷ is selected from the group consisting of hydrogen, fluoride, chloride, bromide, methyl, trifluoromethyl and methoxy;
in the form shown or in form of the acid or base or in form of a salt, especially a physiologically acceptable salt, or in form of a solvate, especially a hydrate.

In a preferred aspect of the invention the compound according to formula Ia used is characterized in that R⁷ is hydrogen.

In a preferred aspect of the invention the compound according to formula la used is characterized in that R⁴ is Methyl.

In a preferred aspect of the invention the compound according to formula la used is characterized in that R⁵ and R⁶ are either hydrogen, CH₃ or C₂H₅ or together with the Nitrogen form a piperidinyl, morpholinyl or pirrolidinyl ring.

In a preferred aspect of the invention the compound according to formula la used is selected from among a group consisting of:
5-{α-[2-(dimethylamino)ethoxy]benzyl}-1-methyl-1*H*-pirazole,
(±)-5-{α-[2-(dimethylamino)ethoxy]benzyl)-1-methyl-1*H*-pirazole,
(+)-5-{α-[2-(dimethylamino)ethoxy]benzyl}-1-methyl-1*H*-pirazole,
(-)-5-{α-[2-(dimethylamino)ethoxy]benzyl}-1-methyl-1*H*-pirazole,
2-((1-methyl-1H-pyrazol-5-yl)(phenyl)methoxy)ethanamine,
2-((1-methyl-1H-pyrazol-5-yl)(phenyl)methoxy)-N-methylethanamine,
5-{α-[2-(dimethylamino)ethoxy]-2-thienylmethyl}-1-methyl-1*H*-pirazole,
(±)-5-{α-[2-(dimethylamino)ethoxy]-2-thienylmethyl}-1-methyl-1*H*-pirazole,
(+)-5-{α-[2-(dimethylamino)ethoxy]-2-thienylmethyl}-1-methyl-1*H*-pirazole,
(-)-5-{α-[2-(dimethylamino)ethoxy]-2-thienylmethyl}-1-methyl-1*H*-pirazole;
2-((1-methyl-1H-pyrazol-5-yl)(thiophen-2-yl)methoxy)ethanamine,
2-((1-methyl-1H-pyrazol-5-yl)(thiophen-2-yl)methoxy)-N-methylethanamine.

In a preferred aspect of the invention the compound according to formula I and la used is a compound of general formula (Ib) 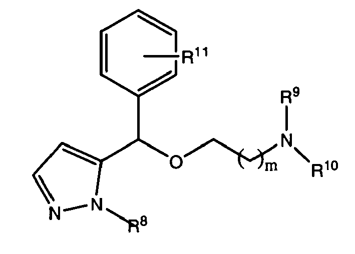
in which
m is 1 or 2;
R⁸ is selected from hydrogen, fluoride, chloride, bromide and methyl;
R⁹ and R¹⁰ are independently selected from hydrogen, lower C(₁₋₄)-Alkyl or together with the Nitrogen form an azaheterocyclic ring;
R¹¹ is selected from the group consisting of hydrogen, fluoride, chloride, bromide, methyl, trifluoromethyl and methoxy;
in the form shown or in form of the acid or base or in form of a salt, especially a physiologically acceptable salt, or in form of a solvate, especially a hydrate.

In a preferred aspect of the invention the compound according to formula Ib used is characterized in that R¹¹ is hydrogen.

In a preferred aspect of the invention the compound according to formula Ib used is characterized in that R⁸ is Methyl.

In a preferred aspect of the invention the compound according to formula Ib used is characterized in that R⁹ and R¹⁰ are either hydrogen, CH₃ or C₂H₅ or together with the Nitrogen form a piperidinyl, morpholinyl or pirrolidinyl ring;
preferably in which R⁹ and R¹⁰ are either hydrogen, CH₃ or C₂H₅;
especially in which R⁹ and R¹⁰ are equal and either CH₃ or C₂H₅;
most preferably in which R⁹ and R¹⁰ are both CH₃.

In a preferred aspect of the invention the compound according to formula Ib used is characterized in that m is 1.

In a preferred aspect of the invention the compound according to formula Ib used is selected from among a group consisting of:
5-{α-[2-(dimethylamino)ethoxy]benzyl}-1-methyl-1*H*-pirazole,
(±)-5-{α-[2-(dimethylamino)ethoxy]benzyl}-1-methyl-1*H*-pirazole,
(+)-5-{α-[2-(dimethylamino)ethoxy]benzyl}-1-methyl-1*H*-pirazole,
(-)-5-{α-[2-(dimethylamino)ethoxy]benzyl}-1-methyl-1*H*-pirazole;
2-((1-methyl-1H-pyrazol-5-yl)(phenyl)methoxy)-N-methylethanamine,
preferably
5-{α-[2-(dimethylamino)ethoxy]benzyl}-1-methyl-1*H*-pirazole,
especially the citrate.

In a preferred aspect of the invention the compound according to formula la used is a compound of general formula (Ic) 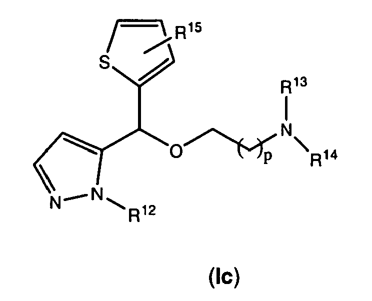
in which
p is 1 or 2;
R¹² is selected from hydrogen, fluoride, chloride, bromide and methyl;
R¹³ and R¹⁴ are independently selected from hydrogen, lower C₍₁₋₄₎-Alkyl or together with the Nitrogen form an azaheterocyclic ring;
R¹⁵ is selected from the group consisting of hydrogen, fluoride, chloride, bromide, methyl, trifluoromethyl and methoxy.

In a preferred aspect of the invention the compound according to formula Ic used is characterized in that R¹⁵ is hydrogen.

In a preferred aspect of the invention the compound according to formula Ic used is characterized in that R¹² is Methyl.

In a preferred aspect of the invention the compound according to formula Ic used is characterized in that R₁₃ and R₁₄ are either hydrogen, CH₃ or C₂H₅ or together with the Nitrogen form a piperidinyl, morpholinyl or pirrolidinyl ring;
preferably in which R₁₃ and R₁₄ are either CH₃ or C₂H₅;
especially in which R₁₃ and R₁₄ are equal and either CH₃ or C₂H₅;
most preferably in which R₁₃ and R₁₄ are both CH₃.

In a preferred aspect of the invention the compound according to formula Ic used is characterized in that p is 1.

In a preferred aspect of the invention the compound according to formula Ic used is selected from among a group consisting of:
5-{α-[2-(dimethylamino)ethoxy]-2-thienylmethyl}-1-methyl-1*H*-pirazole,
(±)-5-{α-[2-(dimethylamino)ethoxy]-2-thienylmethyl}-1-methyl-1*H*-pirazole,
(+)-5-{α-[2-(dimethylamino)ethoxy]-2-thienylmethyl}-1-methyl-1*H*-pirazole,
(-)-5-{α-[2-(dimethylamino)ethoxy]-2-thienylmethyl}-1-methyl-1*H*-pirazole; 2-((1-methyl-1H-pyrazol-5-yl)(thiophen-2-yl)methoxy)-N-methylethanamine.
preferably
5-{α-[2-(dimethylamino)ethoxy]-2-thienylmethyl}-1-methyl-1*H*-pirazole,
especially the citrate.

Unless otherwise stated, the compound of the invention are also meant to include compounds which differ only in the presence of one or more isotopically enriched atoms. For example, compounds having the present structures except for the replacement of a hydrogen by a deuterium or tritium, or the replacement of a carbon by ¹³C- or ¹⁴C-enriched carbon or ¹⁵N-enriched nitrogen are within the scope of this invention.

A preferred use according to the invention is characterized in that the active compound is used in the medicament at a dose between 50 and 400 mg/day or between 200 and 600 mg/day.

In the context of this invention - if not clearly expressed as meaning the complete salt - dose means the dose of the active compound without the salt (which means without the counter ion, for example the citrate ion).

Generally an effective administered amount of a compound used according to the invention will depend on the relative efficacy of the compound chosen, the severity of the disorder being treated and the weight of the sufferer. However, active compounds will typically be administered once or more times a day for example 1, 2, 3 or 4 times daily, with typical total daily doses in the range of from 0.1 to 1000/mg/kg/day.

Any formulation or pharmaceutical composition according to the invention contains the active ingredient as well as optionally at least one auxiliary material and/or additive.

The auxiliary material and/or additive can be selected from carrier, excipient, support materials, glidants, fillers, solvents, diluents, colorants, taste conditioners like sugars, antioxidants and/or binders. In the case of a suppository this might involve waxes or fatty acid esters or conserving agents, emulsifiers and/or carriers for parenteral application. The selection of these auxiliary materials and/or additives and of the amounts to be used depends upon how the pharmaceutical composition is to be applied.

Examples include here oral or parenteral like pulmonal, nasal, rectal and/or intravenous application. Therefore the pharmaceutical composition according to the invention can be adapted for topical or systemical application, especially dermal, subcutaneous, intramuscular, intra-articular and/or intraperitoneal, pulmonal, buccal, sublingual, nasal, percutaneous, vaginal, oral or parenteral, pulmonal, nasal, rectal and/or intravenous application.

For oral application preparations in the form of tablets, chewable tablets, dragees, capsules, granules, drops, juices and syrups are suitable. Solutions, suspensions, readily reconstitutable dry preparations and sprays are suitable i.a. for parenteral application. The compounds according to the invention as a deposit in a dissolved form or in a patch, optionally with the addition of agents which promote dermal penetration, are examples of suitable percutaneous forms of application. Dermal applications include i.a. an ointment, a gel, a cream, a lotion, a suspension, an emulsion whereas the preferred form for rectal application is a suppository.

Solid oral compositions (which are preferred as are liquid ones) may be prepared by conventional methods of blending, filling or tabletting. Repeated blending operations may be used to distribute the active agent throughout those compositions employing large quantities of fillers. Such operations are conventional in the art. The tablets may for example be prepared by wet or dry granulation and optionally coated according to the methods well known in normal pharmaceutical practice., in particular with an enteric coating.

The mentioned formulations will be prepared using standard methods such as those described or referred to in the Spanish and US Pharmacopeias and similar reference texts.

A preferred use according to the invention is characterized in that the medicament is for oral administration, especially in form of a tablet or capsule.

Another use according to the invention is characterized in that the medicament is in form of an immediate release formulation.

In the context of this invention "immediately release formulation" means any formulation with a release profile from which measured according to a standard measurement (e.g. using the paddle method according to the Pharmacopeia) (e.g. in 0.1 % NaCl solution) within 30 minutes more than 50 %, more preferably 60 %, or even more preferably 70 % of the active compound is released.

Another use according to the invention is characterized in that the medicament is in form of a formulation comprising any of the following:
- sodium croscarmelose,
- starch,
- colloidal silica dioxide,
- a salt with stearic acid, especially magnesium stearate,
- povidone,
- microcrystalline cellulose
- lactose monohydrate
- polyethylene glycol.

In a preferred embodiment of the invention the use is characterized in that the medicament manufactured is for drug addiction treatment from addiction to opioids.

In a preferred embodiment of the invention the use is characterized in that the medicament manufactured is for the treatment of drug abuse of opioids

In a preferred embodiment of the invention the use is characterized in that the medicament manufactured is for for the treatment of dependency from opioids

In a preferred embodiment of the invention the use is characterized in that the medicament manufactured is for for de-addiction treatment from addiction to opioids,

In a preferred embodiment of the invention the use is characterized in that the medicament manufactured is for the amelioration of the effects of a de-addiction treatment from opioids.

In a preferred embodiment of the invention the opioid is opium (which is the dried milky juice of the opium poppy containing opioids (alkaloids)), morphine or heroine or any of its analogues and salts.

Included in this invention are especially also methods of treatments of a patient or a mammal, including men, suffering from opioid addiction using the compounds described as being used according to this invention.

The examples and figures in the following section describing pharmacological trials are merely illustrative and the invention cannot be considered in any way as being restricted to these applications.

### Examples:

### Example 1

(±)-5-{α-[2-(dimethylamino)ethoxy]benzyl}-1-methyl-1*H*-pirazole or (±)-5-{α-[2-(dimethylamino)ethoxy]benzyl}-1-methyl-1*H*-pirazole citrate of the formula 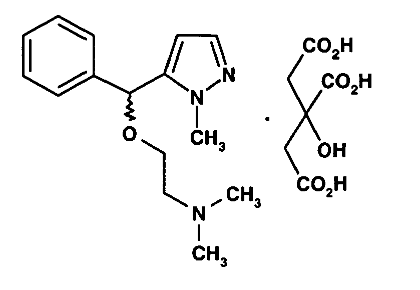 is already described in US 5,017,596 and EP 289 380 B1 including its synthesis and analgetic properties. This compound is also known as cizolirtine.

### Example 2:

### Example of formulation for an injectable (im/iv) solution:

| | |
|---|---|
| Citrate of (±)-5-{α-[2-(dimethylamino)ethoxy]benzyl}-1-methyl-1*H*-pirazole | 50 mg |
| 0.1 N Sodium hydroxide | c.s. pH 6 |
| Water for injection c.s.p. | 1 ml |

### Example 3:

### Example of a formulation (A) for a tablet

| | |
|---|---|
| (±)-5-{α-[2-(dimethylamino)ethoxy]benzyl}-1-methyl-1*H*-pirazole citrate | 400 mg |
| Sodium croscarmelose (Ac-Di-Sol) | 32 mg |
| Colloidal silica dioxide (Aerosyl 200) | 8 mg |
| Magnesium stearate, NF | 16 mg |
| Povidone K-30 | 40 mg |
| Microcrystalline cellulose (Avicel PH-102) | 146 mg |
| Lactose monohydrate (Farmatose 200M) | 158 mg |
| Total | 800 mg |

### Example 4:

### Example of a formulation (B) for a tablet

| | |
|---|---|
| (±)-5-{α-[2-(dimethylamino)ethoxy]benzyl}-1-methyl-1*H*-pirazole citrate | 200 mg |
| Sodium croscarmelose (Ac-Di-Sol) | 32 mg |
| Colloidal silica dioxide (Aerosyl 200) | 8 mg |
| Magnesium stearate, NF | 16 mg |
| Povidone K-30 | 40 mg |
| Microcrystalline cellulose (Avicel PH-102) | 246 mg |
| Lactose monohydrate (Farmatose 200M) | 258 mg |

### Example 5:

### Example of a formulation (C) for a tablet

| | |
|---|---|
| (±)-5-{α-[2-(dimethylamino)ethoxy]benzyl}-1-methyl-1*H*-pirazole citrate | 400 mg |
| Sodium croscarmelose (Ac-Di-Sol) | 35 mg |
| Colloidal silica dioxide (Aerosyl 200) | 3 mg |
| Sodium stearic fumarate | 12 mg |
| Polyethylene glycol 8000 | 30 mg |
| Microcrystalline cellulose (Avicel PH-102) | 75 mg |
| Lactose monohydrate (Farmatose 200M) | 45 mg |
| Total | 600 mg |

### Example 6:

### Example of a formulation of a capsule

| | |
|---|---|
| (±)-5-{α-[2-(dimethylamino)ethoxy]benzyl}-1-methyl-1*H*-pirazole citrate | 200.0 mg |
| Colloidal silica dioxide | 0.8 mg |
| Magnesium stearate | 2.4 mg |
| Lactose | 276.8 mg |
| Total | 480 mg |

### Example 7:

### Determination of withdrawal symptoms

The effect of the inventive active substance in combination with an opioid on withdrawal symptoms after treatment with an opoid is determined according to the publication of J.K. Saelens et al. Int. Pharmacodyn. 1971, 190, pages 213-218 in male Swiss albino mice (weight 20-25 g, Harlan Iberica, S. Feliu de Codinas, Barcelona, Spain).

Opioid dependency is delevoped in the mice by intraperitoneal administration of the opioid in a suitable dose known to those skilled in the art, e.g. 5 mg/kg/day for 4 consecutive days for morphine. Withdrawal symptoms are then induced by the intravenous administration of a suitable opioid antagonist in a dose known to those skilled in the art, for example, 2 mg/kg naloxone in case of morphine, 30 minutes after the administration of the final dose of the opioid is completed.

In the 30 minute period following the naloxone administration the mice show typical withdrawal symptoms, namely jumps and shakes (of the wet dog shake type), which are counted and registered.

The active substance is administered in combination with the opioid to the mice for 4 consecutive days. After the administration of 2 mg/kg naloxone 30 minutes after the administration of the final dose of the active substance in combination with the opioid has been completed, the mice are closely watched for withdrawal symptoms, namely jumps and shakes (of the wet dog shake type), which are then counted and registered.

The influence of the active substance in combination with an opioid on withdrawal symptons has been determined as described above. The results are given in Table A:

**Table A:**

| Preparation administered | Dosis (mg/kg/day) over 4 days | mode of administration | Number of Jumps | Number of shakes |
|---|---|---|---|---|
| vehicle¹ | --- | i.p. | 0 | 0 |
| Morphine | 5 | s.c. | 6.6 | 16.3 |
| Cizorlitine² + Morphine | 40 + 5 | i.p. s.c. | 3.1 | 3.4 |

| | | | | |
|---|---|---|---|---|
| 1: 5 % by weight arabic gum in water for injection purposes | | | | |
| 2: as Citrate salt | | | | |
| i.p.: intraperitoneal | | | | |
| s.c. subcutaneous | | | | |

As can be seen from the values given in table A the withdrawal symptoms usually associated with the administration of opoids (here morphine) are significantly reduced by its co-administration with a substituted carbinol compound - example 1 - Cizolirtin Citrate, even if the administered amount of the opioid is not reduced.

## Claims

**1.** Use of a carbinol compound of general formula (II) 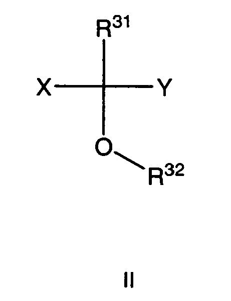
wherein
R³¹ represents a hydrogen atom, a linear or branched alkyl radical, a linear or branched alkenyl radical, an optionally at least mono-substituted cycloaliphatic radical, which may contain at least one nitrogen atom as ring member, or a phenyl radical,
R³² represents a hydrogen atom, an optionally at least one nitrogen atom as ring member containing cycloaliphatic radical, which may be at least mono-substituted by a linear or branched alkyl radical and/or which may be bound via a linear or branched alkylene group, an NR³³R³⁴⁻moiety, which is bound via a linear or branched alkylene group, or an NR³⁵R³⁶-moiety, which is bound via a linear or branched alkylene group,
R³³ and R³⁴, identical or different, represent hydrogen, a linear or branched alkyl radical or an unsubstituted benzyl radical,
R³⁵ and R³⁶ together with the bridging nitrogen atom represent a saturated, unsubstituted, optionally at least one further heteroatom as ring member containing heterocyclic radical,
X represents an optionally at least mono-substituted phenyl radical or an optionally at least mono-substituted thienyl radical, wherein in each case the substituents may be independently selected from the group consisting of a linear or branched alkyl radical, a linear or branched alkoxy group, a linear or branched alkyl radical, which is at least partially halogenated and a halogen atom,
Y represents a heteroaryl radical, which contains one or more nitrogen atoms as ring members and which is not substituted or at least mono-substituted by one or more substitutents independently from one another selected from the group consisting of a halogen atom, a linear or branched alkyl radical, a benzyl radical, a ciano group bound via a linear or branched C₁₋₄-alkylene group, a carboxy group bound via a linear or branched C₁₋₄-alkylene group, a methoxy carbonyl group bound via a linear or branched C₁₋₄-alkylene group, a hydroxy group bound via a linear or branched C₁₋₄-alkylene group, an amino group bound via a linear or branched C₁₋₄-alkylene group, a (C₁₋₄) dialkylamino group bound via a linear or branched C₁₋₄-alkylene group, and a cycloaliphatic radical, which contains at least one nitrogen atom as ring member and which is bound via a linear or branched C₁₋₄-alkylene group, or Y represents an unsubstituted heteroaryl radical, which contains two nitrogen atoms as ring members and which is condensed with a saturated, one methyl-substituted nitrogen atom as ring member containing cycloaliphatic group,
optionally in form of one of its stereoisomers, preferably enantiomers or diastereomers, its racemate or in form of a mixture of at least two of its stereoisomers, preferably enantiomers and/or diastereomers, in any mixing ratio, or a corresponding salt thereof, or a corresponding solvate
for the manufacture of a medicament for drug addiction treatment from addiction to opioids, for the treatment of drug abuse of opioids, for the treatment of dependency from opioids or to ameliorate the effects of a de-addiction treatment from addiction to opioids.

**2.** Use according to claim 1, **characterized in that** R³¹ represents a hydrogen atom, a linear or branched C₁₋₄ alkyl radical, a linear or branched C₂₋₄ alkenyl radical, a 5- or 6-membered cycloaliphatic radical, which may contain at least one nitrogen atom as ring member and/or which may be at least mono-substituted by a linear or branched C₁₋₄ alkyl radical, or a phenyl radical, preferably a hydrogen atom, a linear or branched C₁₋₄ alkyl radical, a vinyl group, a cyclohexyl radical, an N-Methyl-piperidyl radical or a phenyl radical.

**3.** Use according to claim 1 or 2, **characterized in that** R³² represents a hydrogen atom, an optionally at least one nitrogen atom as ring member containing, 5- or 6-membered cycloaliphatic radical, which may be at least mono-substituted by a linear or branched C₁₋₄-alkyl radical and/or which may be bound via a linear or branched C₁₋₄-alkylene group, a NR³³R³⁴-moiety, which is bound via a linear or branched C₁₋₄ alkylene group, or a NR³⁵R³⁶⁻moiety, which is bound via a linear or branched C₁₋₄ alkylene group, preferably a hydrogen atom, an optionally at least one nitrogen atom as ring member containing, 5- or 6-membered cycloaliphatic radical, which may be at least mono-substituted by a linear or branched C₁₋₄-alkyl radical and/or which may be bound via a linear or branched C₁₋₄-alkylene group, a NR³³R³⁴⁻moiety, which is bound via a linear or branched C₂₋₃ alkylene group, or a NR³⁵R³⁶-moiety, which is bound via a linear or branched C₂₋₃ alkylene group.

**4.** Use according to one or more of claims 1-3, **characterized in that** R³³ and R³⁴, identical or different, independently from one another represent hydrogen; a linear or branched C₁₋₄ alkyl radical or an unsubstituted benzyl radical, preferably hydrogen or a linear or branched C₁₋₄ alkyl radical.

**5.** Use according to one or more of claims 1-4, **characterized in that** R³⁵ and R³⁶ together with the bridging nitrogen atom represent a saturated, unsubstituted, optionally at least one oxygen atom as ring member containing, 5- or 6-membered heterocyclic radical.

**6.** Use according to one or more of claims 1-5, **characterized in that** X represents an optionally at least mono-substituted phenyl radical or an optionally at least mono-substituted thienyl radical, wherein in each case the substituents may be independently selected from the group consisting of a linear or branched C₁₋₄ alkyl radical, a linear or branched C₁₋₄ alkoxy radical, a linear or branched C₁₋₄ alkyl radical, which is at least partially fluorinated, a fluorine atom, a chlorine atom and a bromine atom, preferably represents an optionally at least mono-substituted phenyl radical or an optionally at least mono-substituted thienyl radical, wherein in each case the substituents may be independently selected from the group consisting of a methyl radical, a methoxy radical, a trifluoromethyl radical, a fluorine atom, a chlorine atom and a bromine atom.

**7.** Use according to one or more of claims 1-6, **characterized in that** Y represents an azole radical selected from the group consisting of
a) a pyrazole of the general formula (a): 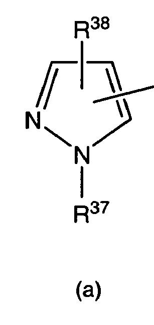 in which R³⁷ represents a linear or branched C₁₋₁₂ alkyl radical, a benzyl radical or a radical of the type: 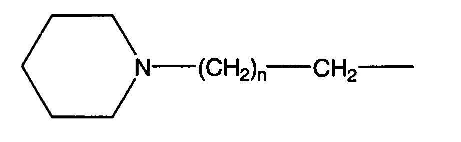 in which n = 1 or 2, and
R³⁸ represents a hydrogen atom, a methyl radical or a halogen atom, preferably a hydrogen atom, a methyl radical, a bromine atom or a chlorine atom,
b) an imidazole of the general formula 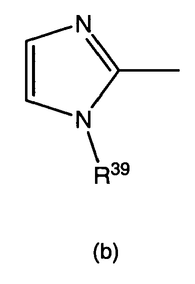 in which R³⁹ represents a hydrogen atom, a C₁-₁₂ alkyl radical, a benzyl radical, or a radical of the general formula (b1):
R⁴⁰-(CH₂)ₙ- **(b1)**
in which n is 2, 3 or 4 and R⁴⁰ represents a piperidinyl radical, a phenyl radical, a cyano group, a hydroxyl radical, a carboxy radical, an amino group, a dimethylamino group, or a methyl ester (CH₃-O-C(=O)-) group,
and
(d) an imidazole of the following formula:

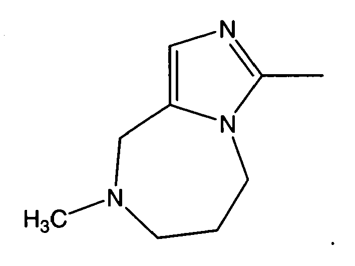

**8.** Use according to one or more of claims 1-7, **characterized in that** at least the one administered compound is a carbinol compound of general formula II 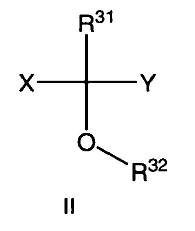 is present, wherein
R³¹ represents a hydrogen atom, a methyl radical, an ethyl radical, an n-propyl radical, an iso-propyl radical, a sec-butyl radical, a tert-butyl radical, an n-butyl radical, a vinyl radical, a cyclohexyl radical, an N-methyl-piperidinyl group, or a phenyl group,
R³² represents a hydrogen atom, a monomethylaminoethyl group, a dimethylaminoethyl group, an aminoethyl group, a pyrrolidinylethyl group, a piperidinylethyl group, a methyl-benzyl-aminoethyl group, a morpholinylethyl group, a diisopropylaminoethyl group, a dimethylaminopropyl group, a piperidinylpropyl group, a pyrrolidinylpropyl group, a morpholinylpropyl group, an N-methyl-2-piperidyl group, an N-ethyl-2-piperidyl group, an N-propyl-2-piperidyl group, an N-methyl-2-pyrrolidinyl group, an N-ethyl-2-pyrrolidinyl group, an N-propyl-2-pyrrolidinyl group, or a 2-dimethylaminoethyl-1-methyl group,
X represents a phenyl radical, a 2-methyl-phenyl radical, a 3-methyl-phenyl radical, a 4-methyl phenyl radical, a 2-chloro-phenyl radical, a 3-chloro-phenyl radical, a 4-chloro-phenyl radical, a 2-fluoro-phenyl radical, a 3-fluoro-phenyl radical, a 4-fluoro-phenyl radical, a 2-trifluoromethyl-phenyl radical, a 3-trifluoromethyl-phenyl radical, a 4-trifluoromethyl-phenyl radical, a 2-methoxy-phenyl radical, a 3-methoxy-phenyl radical, a 4-methoxy-phenyl radical, a 3,4,5-tris-methoxy phenyl radical, a 3,4-dichloro-phenyl radical, a 2,4-dichlorophenylradical, a thien-2-yl radical, a thien-3-yl radical, a 3-methyl-thien-2-yl radical, a 5-methyl-thien-2-yl-radical, a 5-bromo-thien-2-yl radical or a 4-bromothien-2-yl-radical,
Y represents an azole radical selected from the group consisting of
a) a pyrazole of the general formula (a): 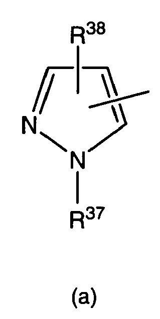 in which
R³⁷ represents a methyl radical, an ethyl radical, an n-propyl radical, an isopropyl radical, an n-butyl radical, a sec-butyl radical or a tert-butyl radical,
R³⁸ represents a hydrogen atom, a methyl radical, a bromine atom or a chlorine atom,
b) an imidazole of the general formula 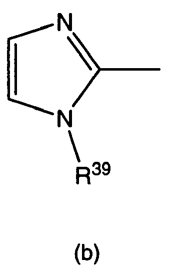 in which R³⁹ represents a hydrogen atom, a methyl radical, an ethyl radical, an n-propyl radical, an iso-butyl radical, an n-butyl radical, a sec-butyl radical a tert-butyl radical, an n-pentyl radical, an n-hexyl radical, an n-heptyl radical, an n-octyl radical, an n-nonyl radical, an n-decyl radical, an n-undecyl radical an n-dodecyl radical, a benzyl radical, or a radical of the general formula (b1):
R⁴⁰-(CH₂)ₙ- (b1)
in which n is 2, 3 or 4 and R⁴⁰ represents a piperidinyl radical, a phenyl radical, a cyano group, a hydroxyl radical, a carboxy radical, an amino group, a dimethylamino group, or a methyl ester group,
and
(c) an imidazole of the following formula: 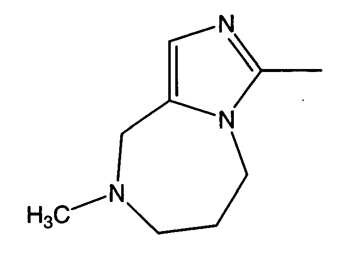 optionally in form of one of its stereoisomers, preferably enantiomers or diastereomers, its racemate or in form of a mixture of at least two of its stereoisomers, preferably enantiomers and/or diastereomers, in any mixing ratio, or a corresponding salt thereof, or a corresponding solvate.

**9.** Use according to one or more of claims 1-8, **characterised in that** at least one administered component is selected from the group consisting of
[212] 2-{α-[2-(dimethylamino)ethoxy]benzyl}-1-methyl-1*H*-imidazole,
[213] 2-{4-chloro-α-[2-(dimethylamino)ethoxy]-α-methylbenzyl}-1-methyl-1*H*- imidazole,
[214] 2-{4-chloro-α-[2-(dimethylamino)ethoxy]benzyl}-1-methyl-1*H-*imidazole,
[215] 2-{3-chloro-α-[2-(dimethylamino)ethoxy]benzyl}-1-methyl-1*H-*imidazole,
[216] 2-{4-chloro-α-[2-(dimethylamino)ethoxy]-α-methylbenzyl}-1-methyl-1*H*- imidazole,
[217] 2-{4-fluoro-α-[2-(dimethylamino)ethoxy]-α-methylbenzyl}-1-methyl-1*H*- imidazole,
[218] 2-{α-[2-(dimethylamino)ethoxy]-α-methyl-3-(trifluoromethyl)benzyl}-1-methyl- 1*H*-imidazole,
[219] 2-{3-chloro-α-[2-(dimethylamino)ethoxy]-α-methylbenzyl}-1-methyl-1 *H*- imidazole,
[220] 2-{3-chloro-α-[2-(dimethylamino)ethoxy]-α-propylbenzyl}-1-methyl-1*H*- imidazole,
[221] 1-butyl-2-{4-chloro-α-[2-(dimethylamino)ethoxy]-α-methylbenzyl}-1*H*-imidazole,
[222] 2-{α-[2-(dimethylamino)ethoxy]-α-methyl-4-methoxybenzyl}-1-methyl-1*H*-imidazole,
[223] 2-{3-chloro-α-methyl-α-[2-(N-pyrrolidinyl)ethoxy]benzyl}-1-methyl-1*H*-imidazole,
[224] 2-{α-[2-(dimethylamino)ethoxy]-α-propyl-3,4,5-trimethoxybenzyl}-1-dodecyl-1*H*-imidazole,
[225] 1-butyl-2-{α-[2-(dimethylamino)ethoxy]-4-(trifluoromethyl)benzyl}-1*H*-imidazole,
[226] 1-methyl-2-{α-methyl-α-[2-(N-piperidyl)ethoxy]-3-(trifluoromethyl)benzyl}-1*H*-imidazole,
[227] 2-{α-cyclohexyl-3,4-dichloro-α-[2-(dimethylamino)ethoxy]benzyl}-1-methyl-1*H*-imidazole,
[228] 2-{3,4-dichloro-α-[2-(dimethylamino)ethoxy]-α-propylbenzyl}-1-methyl-1*H*-imidazole,
[229] 2-{3,4-dichloro-α-[2-(dimethylamino)ethoxy]-α-methylbenzyl}-1-methyl-1*H*-imidazole,
[230] 2-{3,4-dichloro-α-[2-(dimethylamino)ethoxy]benzyl}-1-methyl-1*H*-imidazole,
[231] 2-{4-chloro-α-[2-(dimethylamino)ethoxy]-α-methylbenzyl}-1-[2-(N-piperidyl)ethyl]-1*H*-imidazole,
[232] 2-{4-chloro-α-[2-(dimethylamino)ethoxy]-α-methylbenzyl}-1-[2-(N-piperidyl)propyl]-1*H*-imidazole,
[233] 1-(3-cyanopropyl)-2-{4-chloro-α-[2-(dimethylamino)ethoxy]benzyl}-1*H*-imidazole,
[234] 2-{4-chloro-α-[2-(dimethylamino)ethoxy]-α-(N-methyl-4-piperidyl)benzyl}-1-methyl-1*H*-imidazole,
[235] 1-benzyl-2-{α-[2-(N-benzyl-N-methylamino)ethoxy]-4-chlorobenzyl}-1*H*-imidazole,
[236] 2-{4-chloro-α-[2-(dimethylamino)ethoxy]-α-methylbenzyl}-7-methyl-6,7,8,9-tetrahydro-1*H*-imidazole[1,5-a][1,4]diazepine,
[237] 2-{4-chloro-α-[2-(dimethylamino)ethoxy]benzyl}-7-methyl-6,7,8,9-tetrahydro-1*H*-imidazole[1,5-a][1,4]diazepine,
[238] 1-butyl-5-{α-[2-(dimethylamino)ethoxy]benzyl}-1*H*-pyrazole,
[239] 5-{α-(4-chlorophenyl)-α-[2-(dimethylamino)ethoxy]benzyl}-1-methyl-1*H*-pyrazole,
[240] 1-butyl-5-{α-[2-(dimethylamino)ethoxy]-3,4,5-trimethoxybenzyl}-1*H*-pyrazole,
[241] 1-butyl-5-{4-chloro-α-[2-(dimethylamino)ethoxy]-α-methylbenzyl}-1*H*-pyrazole,
[242] 5-{α-[2-(dimethylamino)ethoxy]benzyl}-1-methyl-1*H*-pyrazole,
[243] 5-{α-[2-(dimethylamino)ethoxy]-α-methylbenzyl}-1-methyl-1*H*-pyrazole,
[244] 5-{α-[2-(dimethylamino)ethoxy]-3,4,5-trimethoxybenzyl}-1-methyl-1 *H*-pyrazole,
[245] 1-methyl-5-{α-[2-(N-pyrrolidinyl)ethoxy]benzyl}-1*H*-pyrazole,
[246] 1-methyl-5-{α-[2-(N-morpholinyl)ethoxy]benzyl}-1*H*-pyrazole,
[247] 5-{α-[2-(dimethylamino)ethoxy]-α-methyl-3,4,5-trimethoxybenzyl}-1-methyl-1*H*-pyrazole,
[248] 4-bromo-5-{α-[2-(dimethylamino)ethoxy]benzyl}-1-methyl-1*H-*pyrazole,
[249] 1,3-dimethyl-5-{α-[2-(dimethylamino)ethoxy]-α-methylbenzyl}-1*H*-pyrazole,
[250] 1,3-dimethyl-5-{α-[2-(dimethylamino)ethoxy]benzyl}-1*H-*pyrazole,
[251] 5-{α-[2-(dimethylamino)ethoxy]-2-methylbenzyl}-1-methyl-1*H-*pyrazole,
[252] 4-chloro-5-{4-chloro-α-[2-(dimethylamino)ethoxy]benzyl}-1-methyl-1*H*-pyrazole,
[253] 5-{4-chloro-α-[2-(dimethylamino)ethoxy]benzyl}-1-methyl-1*H-*pyrazole,
[254] 5-{3-chloro-α-[2-(dimethylamino)ethoxy]benzyl}-1-methyl-1*H-*pyrazole,
[255] 5-{α-[2-(dimethylamino)ethoxy]-4-methylbenzyl}-1-methyl-1*H-*pyrazole,
[256] 5-{2-chloro-α-[2-(dimethylamino)ethoxy]benzyl}-1-methyl-1*H-*pyrazole,
[257] 1-methyl-5-{α-[2-(N-piperidyl)ethoxy]benzyl}-1*H*-pyrazole,
[258] 1-methyl-5-{α-[2-(N-propyl-2-piperidyl)ethoxy]benzyl}-1*H-*pyrazole,
[259] 5-{α-[2-(N-ethyl-2-piperidyl)ethoxy]benzyl}-1-methyl-1*H-*pyrazole,
[260] 1-methyl-5-{α-[2-(N-methyl-2-pyrrolidinyl)ethoxy]benzyl}-1*H-*pyrazole,
[261] 5-{α-[2-(diisopropylamino)ethoxy]benzyl}-1-methyl-1*H-*pyrazole,
[262] 1-methyl-5-{α-[2-(N-methyl-2-piperidyl)ethoxy]benzyl}-1*H-*pyrazole,
[263] 2-{4-chloro-α-[3-(dimethylamino)propoxy]-α-methylbenzyl}-1-methyl-1*H*-imidazole,
[264] 2-{3-chloro-α-[3-(dimethylamino)propoxy]benzyl}-1-methyl-1*H-*imidazole,
[265] 2-{4-chloro-α-[3-(dimethylamino)propoxy]-α-ethylbenzyl}-1-methyl-1*H*-imidazole,
[266] 2-{α-butyl-3-chloro-α-[3-(dimethylamino)propoxy]benzyl}-1-methyl-1*H*-imidazole,
[267] 2-{α-cyclohexyl-4-chloro-α-[3-(dimethylamino)propoxy]benzyl}-1-methyl-1*H*-imidazole,
[268] 2-{α-[3-(dimethylamino)propoxy]-4-fluoro-α-methylbenzyl}-1-methyl-1*H*-imidazole,
[269] 2-{α-[3-(dimethylamino)propoxy]-α-methyl-3-(trifluoromethyl)benzyl}-1-methyl-1*H*-imidazole,
[270] 2-{2-chloro-α-[3-(dimethylamino)propoxy]-α-methylbenzyl}-1-methyl-1*H*-imidazole,
[271] 2-{3-chloro-α-[3-(dimethylamino)propoxy]-α-methylbenzyl}-1-methyl-1*H*-imidazole,
[272] 2-{α-[3-(dimethylamino)propoxy]-α-methyl-3,4,5-trimethoxybenzyl}-1-methyl-1*H*-imidazole,
[273] 2-{α-[3-(dimethylamino)propoxy]-α-methyl-4-methoxybenzyl}-1-methyl-1*H*-imidazole,
[274] 2-{4-chloro-α-[3-(dimethylamino)propoxy]benzyl}-1-methyl-1*H-*imidazole,
[275] 2-{α-[3-(dimethylamino)propoxy]-3,4,5-trimethoxybenzyl}-1-methyl-1*H*-imidazole,
[276] 2-{α-[3-(dimethylamino)propoxy]-α-methyl-4-(trifluoromethyl)benzyl}-1-methyl-1*H*-imidazole,
[277] 2-{α-[3-(dimethylamino)propoxy]-3-(trifluoromethyl)benzyl}-1-methyl-1*H*-imidazole,
[278] 2-{α-[3-(dimethylamino)propoxy]-4-(trifluoromethyl)benzyl}-1-methyl-1*H*-imidazole,
[279] 2-{α-[3-(dimethylamino)propoxy]-4-methoxybenzyl}-1-methyl-1*H*-imidazole,
[280] 2-{α-butyl-α-[3-(dimethylamino)propoxy]-3-(trifluoromethyl)benzyl}-1-methyl-1*H*-imidazole,
[281] 1-butyl-2-{4-chloro-α-[3-(dimethylamino)propoxyl-α-methylbenzyl}-1*H*-imidazole,
[282] 1-butyl-2-{α-butyl-α-[3-(dimethylamino)propoxy]-3,4,5-trimethoxybenzyl}-1*H*-imidazole,
[283] 1-butyl-2-{α-butyl-2-chloro-α-[3-(dimethylamino)propoxy]benzyl}-1*H*-imidazole,
[284] 1-butyl-2-{α-butyl-2,4-dichloro-α-[3-(dimethylamino)propoxy]benzyl}-1*H*-imidazole,
[285] 1-butyl-2-{α-[3-(dimethylamino)propoxy]-4-(trifluoromethyl)benzyl}-1*H*-imidazole,
[286] 2-{4-chloro-α-[3-(N-piperidyl)propoxy]benzyl}-1-methyl-1*H-*imidazole,
[287] 1-methyl-2-{α-methyl-α-[3-(N-piperidyl)propoxy]-4-(trifluoromethyl)benzyl}-1*H*-imidazole,
[288] 2-{α-butyl-2-chloro-α-[3-(dimethylamino)propoxy]benzyl}-1-methyl-1*H*-imidazole,
[289] 2-{α-butyl-3,4-dichloro-α-[3-(dimethylamino)propoxy]benzyl}-1-methyl-1*H*-imidazole,
[290] 2-{3,4-dichloro-α-[3-(dimethylamino)propoxy]-α-methylbenzyl}-1-methyl-1*H*-imidazole,
[291] 2-{3,4-dichloro-α-[3-(dimethylamino)propoxy]benzyl}-1-methyl-1*H*-imidazole,
[292] 2-{α-cyclohexyl-3,4-dichloro-α-[3-(dimethylamino)propoxy]benryl}-1-methyl-1*H*-imidazole,
[293] 2-{4-chloro-α-[3-(dimethylamino)propoxy]-α-methylbenzyl}-α-[2-(N-piperidyl) ethyl]-1*H*-imidazole,
[294] 2-{4-chloro-α-[3-(dimethylamino)propoxy]-α-methylbenzyl}-1-[2-(N-piperidyl) propyl]-1*H*-imidazole,
[295] 2-{4-chloro-α-[3-(dimethylamino)propoxy]-α-(N-methyl-4-piperidyl)benzyl}-1-methyl-1*H*-imidazole,
[296] 1-butyl-5-{α-[3-(dimethylamino)propoxy]benzyl}-1*H*-pyrazole,
[297] 1-butyl-5-{4-chloro-α-[3-(dimethylamino)propoxy]-α-methylbenzyl}-1*H*- pyrazole,
[298] 5-{α-[3-(dimethylamino)propoxy]benzyl}-1-methyl-1*H-*pyrazole,
[299] 5-{α-[3-(dimethylamino)propoxy]-α-methylbenzyl}-1-methyl-1*H*-pyrazole,
[300] 1,3-dimethyl-5-{α-[3-(dimethylamino)propoxy]-α-methylbenzyl}-1*H*-pyrazole,
[301] 1,3-dimethyl-5-{α-[3-(dimethylamino)propoxy]benzyl}-1*H-*pyrazole,
[302] 5-{α-[3-(dimethylamino)propoxy]-2-methylbenzyl}-1-methyl-1*H*-pyrazole,
[303] 5-chloro-5-{4-chloro-α-[3-(dimethylamino)propoxy]benzyl}-1-methyl-1*H*-pyrazole,
[304] 1-methyl-5-{α-[3-(N-piperidyl)propoxy]benzyl}-1*H*-pyrazole,
[305] 1-methyl-5-{α-[3-(N-pyrrolidinyl)propoxy]benzyl}-1*H*-pyrazole,
[306] 4-{4-chloro-α-[2-(dimethylamino)ethoxy]benzyl}-1-methyl-1*H-*pyrazole,
[307] 4-{4-chloro-α-[2-(dimethylamino)ethoxy]-α-methylbenzyl}-1-methyl-1*H*-pyrazole,
[308] 4-{4-chloro-α-[2-(N-propyl-2-piperidyl)ethoxy]benzyl}-1-methyl-1*H*-pyrazole,
[309] 4-{4-chloro-α-[2-(N-methyl-2-piperidyl)ethoxy]benzyl}-1-methyl-1*H*-pyrazole,
[310] 4-{4-chloro-α-[2-(N-ethyl-2-piperidyl)ethoxy]benzyl}-1-methyl-1*H*-pyrazole,
[311] 4-{4-chloro-α-[2-(diisopropylamino)ethoxy]benzyl}-1-methyl-1*H*-pyrazole,
[312] 4-{4-chloro-α-[2-(N-methyl-2-pyrrolidinyl)ethoxy]benzyl}-1-methyl-1*H*-pyrazole,
[313] 4-{α-[3-(dimethylamino)propoxy]benzyl}-1-methyl-1*H-*pyrazole,
[314] 4-{4-chloro-α-[3-(N-morpholinyl)propoxy]benzyl}-1-methyl-1*H-*pyrazole,
[315] 4-{4-chloro-α-[3-(N-pyrrolidinyl)propoxy]benzyl}-1-methyl-1*H-*pyrazole,
[316] 2-(α-hydroxybenzyl)-1*H*-imidazole,
[317] 2-(4-chloro-α-hydroxybenzyl)-1*H*-imidazole,
[318] 2-(4-chloro-α-hydroxybenzyl)-1-methyl-1*H*-imidazole,
[319] 2-(3-chloro-α-hydroxybenzyl)-1-methyl-1*H*-imidazole,
[320] 2-(4-fluoro-α-hydroxybenzyl)-1-methyl-1*H*-imidazole,
[321] 2-[α-hydroxy-3-(trifluoromethyl)benzyl]-1-methyl-1*H*-imidazole,
[322] 2-[α-hydroxy-4-(trifluoromethyl)benzyl]-1-methyl-1*H*-imidazole,
[323] 2-(α-hydroxy-3,4,5-trimethoxybenzyl)-1-methyl-1*H*-imidazole,
[324] 2-(3,4-dichloro-α-hydroxybenzyl)-1-methyl-1*H*-imidazole,
[325] 1-butyl-2-[α-hydroxy-4-(trifluoromethyl)benzyl]-1*H*-imidazole,
[326] 1-butyl-2-(3,4-dichloro-α-hydroxybenzyl)-1*H*-imidazole,
[327] 1-butyl-2-(4-chloro-α-hydroxybenzyl)-1*H*-imidazole,
[328] 1-butyl-2-(α-hydroxy-3,4,5-trimethoxybenzyl)-1*H*-imidazole,
[329] 1-dodecyl-2-(α-hydroxy-3,4,5-trimethoxybenzyl)-1*H*-imidazole,
[330] 2-(α-butyl-3-chloro-α-hydroxybenzyl)-1-methyl-1*H*-imidazole,
[331] 2-(3-chloro-α-hydroxy-α-methylbenzyl)-1-methyl-1*H*-imidazole,
[332] 2-(4-chloro-α-hydroxy-α-methylbenzyl)-1-methyl-1*H-*imidazole,
[333] 2-[4-chloro-α-hydroxy-α-(N-methyl-4-piperidyl)benzyl]-1-methyl-1*H*-imidazole,
[334] 2-(4-chloro-α-ethyl-α-hydroxybenzyl)-1-methyl-1*H*-imidazole,
[335] 2-(α-butyl-4-chloro-α-hydroxybenzyl)-1-methyl-1*H*-imidazole,
[336] 2-(α-cyclohexyl-4-chloro-α-hydroxybenzyl)-1-methyl-1*H-*imidazole,
[337] 2-(2-chloro-α-hydroxy-α-methylbenzyl)-1-methyl-1*H*-imidazole,
[338] 2-(α-butyl-2-chloro-α-hydroxybenzyl)-1-methyl-1*H*-imidazole,
[339] 2-[α-hydroxy-α-methyl-3-(trifluoromethyl)benzyl]-1-methyl-1*H*-imidazole,
[340] 2-[α-butyl-α-hydroxy-3-(trifluoromethyl)benzyl]-1-methyl-1*H-*imidazole,
[341] 2-[α-cyclohexyl-α-hydroxy-3-(trifluoromethyl)benzyl]-1-methyl-1*H*-imidazole,
[342] 2-[α-hydroxy-α-methyl-4-(trifluoromethyl)benzyl]-1-methyl-1*H-*imidazole,
[343] 2-(4-fluoro-α-hydroxy-α-methylbenzyl)-1-methyl-1*H*-imidazole,
[344] 2-(α-hydroxy-α-methyl-4-methoxybenzyl)-1-methyl-1*H-*imidazole,
[345] 2-(3,4-dichloro-α-hydroxy-α-methylbenzyl)-1-methyl-1*H-*imidazole,
[346] 2-(α-butyl-3,4-dichloro-α-hydroxybenzyl)-1-methyl-1*H-*imidazole,
[347] 2-(α-cyclohexyl-3,4-dichloro-α-hydroxybenzyl)-1-methyl-1*H-*imidazole,
[348] 2-(α-hydroxy-α-methyl-3,4,5-trimethoxybenzyl)-1-methyl-1*H-*imidazole,
[349] 1-butyl-2-(4-chloro-α-hydroxy-α-methylbenzyl)-1*H*-imidazole,
[350] 1-butyl-2-(α-butyl-4-chloro-α-hydroxybenzyl]-1*H*-imidazole,
[351] 1-butyl-2-[4-chloro-α-hydroxy-α-(N-methyl-4-piperidyl)benzyl]-1*H*-imidazole,
[352] 1-butyl-2-(α-butyl-α-hydroxy-3,4,5-trimethoxybenzyl)-1*H-*imidazole,
[353] 1-butyl-2-(α-butyl-2-chloro-α-hydroxybenzyl)-1*H*-imidazole,
[354] 1-butyl-2-[α-ethyl-α-hydroxy-3-(trifluoromethyl)benzyl]-1*H-*imidazole,
[355] 1-butyl-2-(α-butyl-2,4-dichloro-α-hydroxybenzyl)-1*H*-imidazole,
[356] 2-(4-chloro-α-hydroxy-α-methylbenzyl)-1-[2-(N-piperidyl)ethyl]-1*H*-imidazole,
[357] 2-(4-chloro-α-hydroxy-α-methylbenzyl)-1-(3-dimethylaminopropyl)-1*H*-imidazole,
[358] 2-(α-butyl-α -hydroxy-3,4,5-trimethoxybenzyl)-1-dodecyl-1*H-*imidazole,
[359] 1-benzyl-2-[α-butyl-α-hydroxy-3-(trifluoromethyl)benzyl]-1*H-*imidazole,
[360] 1-benzyl-2-(4-chloro-α-hydroxy-α-methylbenzyl)-1*H*-imidazole,
[361] 1-(2-cyanoethyl)-2-(4-chloro-α-hydroxybenzyl)-1*H*-imidazole,
[362] 1-(3-aminopropyl)-2-(4-chloro-α-hydroxybenzyl)-1*H*-imidazole,
[363] 3-[2-(3-chloro-α-hydroxybenzyl)-1*H*-imidazole-1-yl]propanoic acid,
[364] 2-(4-chloro-α-hydroxybenzyl)-1-(3-hydroxypropyl)-1*H-*imidazole,
[365] 3-[2-(3-chloro-α-hydroxybenzyl)-1*H*-imidazole-1-yl]methylpropanoate,
[366] 2-(α-hydroxybenzyl)-1-(3-hydroxypropyl)-1*H*-imidazole,
[367] 2-(α-hydroxy-4-methylbenzyl)-1-(3-hydroxypropyl)-1*H-*imidazole,
[368] 2-(α-hydroxy-4-methoxybenzyl)-1-(3-hydroxypropyl)-1*H-*imidazole,
[369] 2-(3,4-dichloro-α-hydroxybenzyl)-1-(3-hydroxypropyl)-1*H-*imidazole,
[370] 3-{2-(α-hydroxybenzyl)-1*H*-imidazole-1-yll}-methyl propanoate,
[371] 2-(4-chloro-α-hydroxybenzyl)-1-(4-hydroxybutyl)-1*H*-imidazole,
[372] 1-(3-cyanopropyl)-2-(4-chloro-α-hydroxybenzyl)-1*H*-imidazole,
[373] 4-[2-(4-chloro-α-hydroxybenzyl)-1*H*-imidazole-1-yl]butanoic acid,
[374] 4-[2-(4-chloro-α-hydroxybenzyl)-1*H*-imidazole-1-yl]-methyl butanoate,
[375] 1-butyl-5-(α-hydroxybenzyl)-1*H*-pyrazole,
[376] 5-(4-chloro-α-hydroxybenzyl)-1-methyl-1*H*-pyrazole,
[377] 5-(α-hydroxy-3,4,5-trimethoxybenzyl)-1-methyl-1*H*-pyrazole,
[378] 1-butyl-5-(α-hydroxy-3,4,5-trimethoxybenzyl)-1*H*-pyrazole,
[379] 4-bromo-5-(α-hydroxybenzyl)-1-methyl-1*H*-pyrazole,
[380] 5-[α-(4-chlorophenyl)-α-hydroxybenzyl]-1-methyl-1*H*-pyrazole,
[381] 1-butyl-5-(4-chloro-α-hydroxy-α-methylbenzyl)-1*H*-pyrazole,
[382] 5-(α-hydroxy-α-methylbenzyl)-1-methyl-1*H*-pyrazole,
[383] 5-(α-hydroxy-α-methyl-3,4,5-trimethoxybenzyl)-1-methyl-1*H-*pyrazole,
[384] 1,3-dimethyl-5-(α-hydroxy-α-methylbenzyl)-1*H*-pyrazole,
[385] 1-butyl-5-(α-hydroxy-α-vinylbenzyl)-1*H*-pyrazole,
[386] 1-butyl-5-(4-chloro-α-hydroxy-α-vinylbenzyl)-1*H*-pyrazole,
[387] 4-chloro-5-(α-hydroxybenzyl)-1-methyl-1*H*-pyrazole,
[388] 5-(α-hydroxy-2-methylbenzyl)-1-methyl-1*H*-pyrazole,
[389] 5-(3-chloro-α-hydroxybenzyl)-1-methyl-1*H*-pyrazole,
[390] 5-(α-hydroxy-4-methylbenzyl)-1-methyl-1*H*-pyrazole,
[391] 5-(2-chloro-α-hydroxybenzyl)-1-methyl-1*H*-pyrazole,
[392] 5-(α-hydroxy-4-methoxybenzyl)-1-methyl-1*H*-pyrazole,
[393] 5-{α-[2-(dimethylamino)ethoxy]-2-thienylmethyl}-1-methyl-1*H-*pyrazole,
[394] 5-{α-[2-(dimethylamino)ethoxy]-2-thienylmethyl}-1-methyl-1*H-*pyrazole citrate,
[395] 5-{α-[2-(dimethylamino)ethoxy]-3-thienylmethyl}-1-methyl-1*H-*pyrazole,
[396] 2-{α-[2-(dimethylamino)ethoxy]-2-thienylmethyl}-1-methyl-1*H-*imidazole,
[397] 5-{α-[2-(dimethylamino)ethoxy]-3-methyl-2-thienylmethyl}-1-methyl-1*H*-pyrazole,
[398] 5-{α-[2-(dimethylamino)ethoxy]-5-methyl-2-thienylmethyl}-1-methyl-1*H*-pyrazole,
[399] 5-{5-bromo-α-[2-(dimethylamino)ethoxy]-2-thienylmethyl}-1-methyl-1*H*-pyrazole,
[400] 5-{4-bromo-α-[2-(dimethylamino)ethoxy]-2-thienylmethyl}-1-methyl-1*H*-pyrazole,
[401] 5-{α-[2-(dimethylamino)ethoxy]-α-methyl-2-thienylmethyl}-1-methyl-1*H*-pyrazole,
[402] 5-{α-[2-(dimethylamino)ethoxy]benzyl}-1-methyl-1*H*-pyrazole citrate,
[403] (±)-5-{α-[2-(dimethylamino)-1-(methyl)ethoxy]benzyl}-1-methyl-1*H*-pyrazole,
[404] (±)-5-{α-[2-(dimethylamino)-1-(methyl)ethoxy]benzyl}-1-methyl-1*H*-pyrazole,
[405] (+)-5-{α-[2-(dimethylamino)ethoxy]-2-thienylmethyl}-1-methyl-1*H*-pyrazole,
[406] (-)-5-{α-[2-(dimethylamino)ethoxy]-2-thienylmethyl}-1-methyl-1*H*-pyrazole,
[407] (+)-5-{α-[2-(dimethylamino)ethoxy]-2-thienylmethyl}-1-methyl-1*H*-pyrazole citrate,
[408] (-)-5-{α-[2-(dimethylamino)ethoxy]-2-thienylmethyl}-1-methyl-1*H*-pyrazole citrate,
[409] (+)-5-{α-[2-(dimethylamino)ethoxy]-2-thienylmethyl}-1-methyl-1*H*-pyrazole-D-ditoluyltartrat,
[410] (-)-5-{α-[2-(dimethylamino)ethoxy]-2-thienylmethyl}-1-methyl-1*H*-pyrazole D-ditoluyltartrat,
[411] (+)-5-{α-[2-(dimethylamino)ethoxy]benzyl}-1-methyl-1*H-*pyrazole citrate,
[412] (-)-5-{α-[2-(dimethylamino)ethoxy]benzyl}-1-methyl-1*H-*pyrazole citrate,
[413] 5-(α-hydroxy-2-thienylmethyl)-1-methyl-1*H*-pyrazole,
[414] 5-(α-hydroxy-3-methyl-2-thienylmethyl)-1-methyl-1*H*-pyrazole,
[415] 5-(α-hydroxy-5-methyl-2-thienylmethyl)-1-methyl-1*H*-pyrazole,
[416] 5-(5-bromo-α-hydroxy-2-thienylmethyl)-1-methyl-1*H*-pyrazole,
[417] 5-(4-bromo-α-hydroxy-2-thienylmethyl)-1-methyl-1*H*-pyrazole,
[418] 5-(α-hydroxy-α-methyl-2-thienylmethyl)-1-methyl-1*H*-pyrazole,
[419] 2-((1-methyl-1H-pyrazol-5-yl)(thiophen-2-yl)methoxy)ethanamine,
[420] 2-((1-methyl-1H-pyrazol-5-yl)(thiophen-2-yl)methoxy)-N-methylethanamine,
[421] 2-((1-methyl-1H-pyrazol-5-yl)(phenyl)methoxy)ethanamine
and
[422] 2-((1-methyl-1H-pyrazol-5-yl)(phenyl)methoxy)-N-methylethanamine.

**10.** Use according to any of claims 1 to 9 **characterized in that** a compound of general formula (I) is used 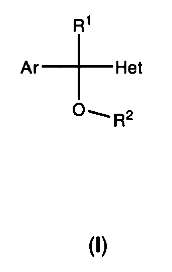 in which
Ar represents a phenyl radical or a thienyl radical, with no substitutions or optionally with 1, 2 or 3 equal or different substituents, selected from a group consisting of fluoride, chloride, bromide, methyl, trifluoromethyl and methoxy;
R¹ represents hydrogen or a lower alkyl group from C₁ to C₄;
R² represents a dialkyl(C₁-C₄)aminoalkyl (C₂-C₃), a monoalkyl(C₁-C₄)aminoalkyl (C₂-C₃), an aminoalkyl (C₂-C₃), or azaheterocyclylalkyl (C₂-C₃) radical; and
Het represents a five-armed nitrogenated aromatic heterocycle that contains one to three nitrogen atoms, without substitutions or optionally substituted by 1 or 2 equal or different substituents selected from a group consisting of fluoride, chloride, bromide and methyl;
optionally in the form of its racemate, pure stereoisomers, especially enantiomers or diastereomers or in the form of mixtures of stereoisomers, especially enantiomers or diastereomers, in any suitable ratio;
in the form shown or in form of the acid or base or in form of a salt, especially a physiologically acceptable salt, or in form of a solvate, especially a hydrate.

**11.** Use according to Claim 10, **characterized in that** R¹ is selected from hydrogen or from a group consisting of methyl, ethyl, propyl, isopropyl, butyl, isobutyl, *sec-*butyl and *tert*-butyl.

**12.** Use according to Claim 10, **characterized in that** R² is selected from among a group consisting of dimethylaminoethyl, dimethylaminopropyl, diethylaminoethyl, diethylaminopropyl, methylaminoethyl, methylaminopropyl, aminoethyl, aminopropyl, piperidinylethyl, piperidinylpropyl, morpholinylpropyl, morpholinylethyl, pirrolidinylpropyl and pirrolidinylethyl;
preferably
dimethylaminoethyl, dimethylaminopropyl, diethylaminoethyl, piperidinylethyl, morpholinylpropyl, and pirrolidinylethyl.

**13.** Use according to Claim 10, **characterized in that** the compound used is a compound of general formula (Ia) 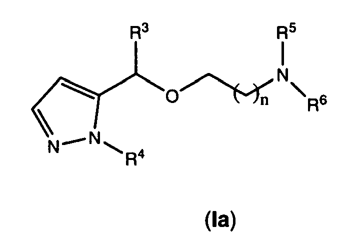 in which
n is 1 or 2;
R³ is selected from: 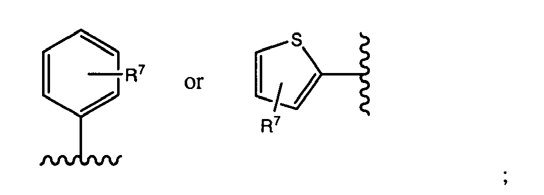
R⁴ is selected from hydrogen, fluoride, chloride, bromide and methyl;
R⁵ and R⁶ are independently selected from hydrogen, lower C₍₁₋₄₎-Alkyl or together with the Nitrogen form an azaheterocyclic ring;
R⁷ is selected from the group consisting of hydrogen, fluoride, chloride, bromide, methyl, trifluoromethyl and methoxy;
in the form shown or in form of the acid or base or in form of a salt, especially a physiologically acceptable salt, or in form of a solvate, especially a hydrate.

**14.** Use according to Claim 13, **characterized in that** R⁷ is hydrogen.

**15.** Use according to Claim 13, **characterized in that** R⁴ is Methyl.

**16.** Use according to Claim 13, **characterized in that** R⁵ and R⁶ are either hydrogen, CH₃ or C₂H₅ or together with the Nitrogen form a piperidinyl, morpholinyl or pirrolidinyl ring.

**17.** Use according to Claim 13, **characterized in that** the compound of general formula (Ia) used is selected from among a group consisting of:
5-{α-[2-(dimethylamino)ethoxy]benzyl}-1-methyl-1*H*-pirazole,
(±)-5-{α-[2-(dimethylamino)ethoxy]benzyl}-1-methyl-1*H*-pirazole,
(+)-5-{α-[2-(dimethylamino)ethoxy]benzyl}-1-methyl-1*H*-pirazole,
(-)-5-{α-[2-(dimethylamino)ethoxy]benzyl}-1-methyl-1*H*-pirazole,
2-((1-methyl-1H-pyrazol-5-yl)(phenyl)methoxy)ethanamine,
2-((1-methyl-1H-pyrazol-5-yl)(phenyl)methoxy)-N-methylethanamine,
5-{α-[2-(dimethylamino)ethoxy]-2-thienylmethyl}-1-methyl-1*H*-pirazole,
(±)-5-{α-[2-(dimethylamino)ethoxy]-2-thienylmethyl}-1-methyl-1*H*-pirazole,
(+)-5-{α-[2-(dimethylamino)ethoxy]-2-thienylmethyl}-1-methyl-1*H*-pirazole,
(-)-5-{α-[2-(dimethylamino)ethoxy]-2-thienylmethyl}-1-methyl-1*H*-pirazole;
2-((1-methyl-1H-pyrazol-5-yl)(thiophen-2-yl)methoxy)ethanamine,
2-((1-methyl-1H-pyrazol-5-yl)(thiophen-2-yl)methoxy)-N-methylethanamine.

**18.** Use according to Claim 13, **characterized in that** it the compound used is a compound of general formula (Ib) 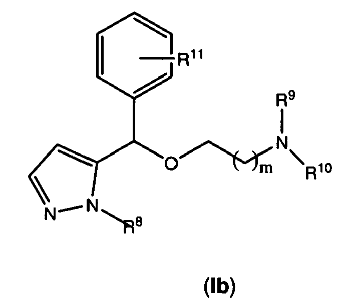 in which
m is 1 or 2;
R⁸ is selected from hydrogen, fluoride, chloride, bromide and methyl;
R⁹ and R¹⁰ are independently selected from hydrogen, lower C₍₁₋₄₎-Alkyl or together with the Nitrogen form an azaheterocyclic ring;
R¹¹ is selected from the group consisting of hydrogen, fluoride, chloride, bromide, methyl, trifluoromethyl and methoxy;
in the form shown or in form of the acid or base or in form of a salt, especially a physiologically acceptable salt, or in form of a solvate, especially a hydrate.

**19.** Use according to Claim 18, **characterized in that** R¹¹ is hydrogen.

**20.** Use according to Claim 18, **characterized in that** R⁸ is Methyl.

**21.** Use according to Claim 18, **characterized in that** R⁹ and R¹⁰ are either hydrogen, CH₃ or C₂H₅ or together with the Nitrogen form a piperidinyl, morpholinyl or pirrolidinyl ring;
preferably in which R⁹ and R¹⁰ are either hydrogen, CH₃ or C₂H₅;
especially in which R⁹ and R¹⁰ are equal and either CH₃ or C₂H₅;
most preferably in which R⁹ and R¹⁰ are both CH₃.

**22.** Use according to Claim 18, **characterized in that** m is 1.

**23.** Use according to Claim 18, **characterized in that** the compound of general formula (Ib) used is selected from among a group consisting of:
5-{α-[2-(dimethylamino)ethoxy]benzyl}-1-methyl-1*H*-pirazole,
(±)-5-{α-[2-(dimethylamino)ethoxy]benzyl}-1-methyl-1*H*-pirazole,
(+)-5-{α-[2-(dimethylamino)ethoxy]benzyl}-1-methyl-1*H*-pirazole,
(-)-5-{α-[2-(dimethylamino)ethoxy]benzyl}-1-methyl-1*H*-pirazole;
2-((1-methyl-1H-pyrazol-5-yl)(phenyl)methoxy)-N-methylethanamine,
preferably
5-{α-[2-(dimethylamino)ethoxy]benzyl}-1-methyl-1*H*-pirazole,
especially the citrate.

**24.** Use according to Claim 13, **characterized in that** the compound used is a compound of general formula (Ic) 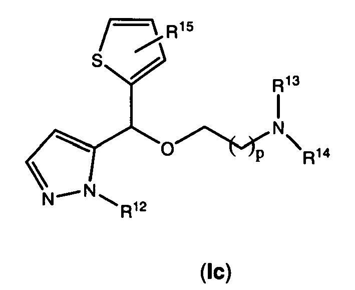 in which
p is 1 or 2;
R¹² is selected from hydrogen, fluoride, chloride, bromide and methyl;
R¹³ and R¹⁴ are independently selected from hydrogen, lower C₍₁₋₄₎-Alkyl or together with the Nitrogen form an azaheterocyclic ring;
R¹⁵ is selected from the group consisting of hydrogen, fluoride, chloride, bromide, methyl, trifluoromethyl and methoxy.

**25.** Use according to Claim 24, **characterized in that** R¹⁵ is hydrogen.

**26.** Use according to Claim 24, **characterized in that** R¹² is Methyl.

**27.** Use according to Claim 24, **characterized in that** R₁₃ and R₁₄ are either hydrogen, CH₃ or C₂H₅ or together with the Nitrogen form a piperidinyl, morpholinyl or pirrolidinyl ring;
preferably in which R₁₃ and R₁₄ are either CH₃ or C₂H₅;
especially in which R₁₃ and R₁₄ are equal and either CH₃ or C₂H₅;
most preferably in which R₁₃ and R₁₄ are both CH₃.

**28.** Use according to Claim 24, **characterized in that** p is 1.

**29.** Use according to Claim 24, **characterized in that** the compound of general formula (Ic) used id selected from among a group consisting of:
5-{α-[2-(dimethylamino)ethoxy]-2-thienylmethyl}-1-methyl-1*H*-pirazole,
(±)-5-{α-[2-(dimethylamino)ethoxy]-2-thienylmethyl)-1-methyl-1*H*-pirazole,
(+)-5-{α-[2-(dimethylamino)ethoxy]-2-thienylmethyl}-1-methyl-1*H*-pirazole,
(-)-5-{α-[2-(dimethylamino)ethoxy]-2-thienylmethyl}-1-methyl-1*H*-pirazole; 2-((1-methyl-1H-pyrazol-5-yl)(thiophen-2-yl)methoxy)-N-methylethanamine.
preferably
5-{α-[2-(dimethylamino)ethoxy]-2-thienylmethyl}-1-methyl-1*H*-pirazole,
especially the citrate.

**35.** Use according to any of claims 1 to 34, **characterized in that** the active compound is used in the medicament at a dose between 50 and 400 mg/day or between 200 and 600 mg/day.

**36.** Use according to any of claims 1 to 35, **characterized in that** the medicament is for oral administration, especially in form of a tablet or capsule.

**37.** Use according to any of claims 1 to 36, **characterized in that** the medicament is in form of a formulation comprising any of the following:
• sodium croscarmelose,
• starch,
• colloidal silica dioxide,
• a salt with stearic acid, especially magnesium stearate,
• povidone,
• microcrystalline cellulose
• lactose monohydrate
• polyethylene glycol.

**38.** Use according to any of claims 1 to 37, **characterized in that** the medicament manufactured is for drug addiction treatment from addiction to opioids.

**39.** Use according to any of claims 1 to 37, **characterized in that** the medicament manufactured is for the treatment of drug abuse of opioids

**40.** Use according to any of claims 1 to 37, **characterized in that** the medicament manufactured is for the treatment of dependency from opioids

**41.** Use according to any of claims 1 to 37, **characterized in that** the medicament manufactured is for de-addiction treatment from addiction to opioids,

**42.** Use according to any of claims 1 to 37, **characterized in that** the medicament manufactured is for the amelioration of the effects of a de-addiction treatment from opioids.

**43.** Use according to any of claims 1 to 42, **characterized in that** the opioid is opium, morphine or heroine or any of its analogues and salts.
